# EUROPEAN PATENT SPECIFICATION

(11) **EP 4 009 988 B1**
(45) Date of publication and mention of the grant of the patent: **20.05.2026**
(21) Application number: 20757843.6
(22) Date of filing: 07.08.2020
(51) Int. Cl.: A61K 33/00, A61K 9/00, A61K 47/12, A61K 47/10, A61P 33/00, A61P 31/12, A61P 31/10, A61P 31/04, A61P 31/02, A61P 31/00, A61P 17/02, A61L 26/00, A61K 35/64, A61K 33/38, A61K 33/34, A61K 33/30, A61K 33/26, A61K 33/06, A61K 33/04

(54) **LIQUID DRESSING COMPOSITIONS COMPRISING SHELLAC AND METALS FOR VETERINARY USES**
FLÜSSIGE VERBANDSZUSAMMENSETZUNGEN MIT SCHELLACK UND METALLEN FÜR VETERINÄRE VERWENDUNGEN
COMPOSITIONS DE PANSEMENT LIQUIDE COMPRENANT DE LA GOMME LAQUE ET DES MÉTAUX POUR UTILISATIONS VÉTÉRINAIRES

(30) Priority: 08.08.2019 GB 201911361; 27.05.2020 GB 202007917
(43) Date of publication of application: 15.06.2022
(73) Proprietor: Cambridge Enterprise Limited, Cambridge, Cambridgeshire CB2 1TN (GB)
(72) Inventor: POWELL, Jonathan, Cambridge Cambridgeshire CB3 0ES (GB); FARIA, Nuno, Cambridge Cambridgeshire CB3 0ES (GB); BASTOS, Carlos Passos, Cambridge Cambridgeshire CB3 0ES (GB); THOM, Will, Cambridge Cambridgeshire CB3 0ES (GB)
(74) Representative: Mewburn Ellis LLP
(86) International application number: PCT/EP2020/072317
(87) International publication number: WO 2021/023886

(56) References cited:
- EP-A1- 0 900 560
- EP-A1- 1 399 128
- EP-A1- 1 679 078
- US-A1- 2010 297 043
- MURPHY SUZANNE ET AL: "Cutaneous Squamous Cell Carcinoma in the Cat : Current understanding and treatment approaches", JOURNAL OF FELINE MEDICINE AND SURGERY, vol. 15, no. 5, 19 April 2013 (2013-04-19), GB, pages 401 - 407, XP093348805, ISSN: 1098-612X, Retrieved from the Internet <URL:https://journals.sagepub.com/doi/pdf/10.1177/1098612X13483238> DOI: 10.1177/1098612X13483238

## Description

### Field of the Invention

The present invention relates to liquid dressing compositions and their veterinary medical uses, in particular for the prevention and/or the treatment of wounds and/or infection. The present invention further relates to devices for delivering or applying the liquid dressing compositions.

### Background of the Invention

There is an unmet need for liquid dressings that are easy to apply, effective, remain sterile and protect lesions and other wounds from external infective agents in a veterinary setting for the treatment of non-human animals. Also, where topical application of antibiotics (or other materials) to lesions is necessary, such liquid dressings should ideally 'lock-in' those materials to their environment and prevent external losses and therefore increase treatment efficacy.

Digital dermatitis (DD) is a hoof infection that affects up to 40% of dairy cows. It is caused by indoor housing of dairy cows, which is needed due to the elevated dietary requirements of high performing milking cows. It is characterised by painful ulcerative or proliferative lesions and causes lameness, reduces milk output and increases mortality rates, with associated economic inefficiency and animal welfare concerns. The treatment of digital dermatitis is a challenging problem as the wounds associated with it are typically chronically infected with multiple species of anaerobic bacteria, especially spirochetes. As the hooves of the animals are often in constant contact with faecal slurry, this also allows disease to spread between cows in a herd and re-infection of previously treated animals.

Existing treatments of digital dermatitis include the application of topical antibiotics or metal-based antimicrobials to the lesions. However, all of these treatments are only modestly effective because the hooves are in constant contact with faecal slurry, leading to the rapid loss of the antimicrobials or antibiotics into the external environment. It is not practical to bandage every time following application of antibiotic or similar treatments to try to prevent their loss from the applied area of the animal into the external environment. Such bandages must anyway be removed after a number of days or they can act negatively as harbingers of infection. Besides financial losses, these repeated cycles of infection and antibiotic application promote the development of antibiotic resistant bacteria, which is a serious public health challenge.

Beyond traditional antibiotics, other antimicrobial treatments include the use of formalin or ionic copper footbaths or the application of copper and zinc EDTA gel. The treatment of heavily infected herds with footbaths can help to control the spread of infection, but does not treat the infected animals. It also has the disadvantage of producing large quantities of environmentally toxic waste, which is expensive to dispose of in a safe manner. Formalin is also carcinogenic. Copper and zinc EDTA gel has limited antimicrobial activity and like antibiotics requires multiple applications, with heavy infections also requiring bandaging. Some formulations of copper and zinc EDTA gel are thick and require application with a brush with the associated risk of cross-infection between treated animals. There is no durable quality of these products beyond reliance upon viscosity so their on-wound lifetime will be limited in such a challenging environment. In view of these disadvantages, it remains a problem in the art to treat digital dermatitis in an effective way.

Antic et al. (Meat Science, 88: 498-502, 2011) describes the application of shellac to cattle hides to limit microbial cross-contamination of beef carcass meat during the skinning operation.

US 2010/0297043 (Lowndes et al) describes cosmetic compositions for coating skin that comprise a natural resin and a non-drying or semi-drying oil as a flexibilizer. EP 0 900 560 A describes film forming compositions for coating teeth and preventing dental caries and periodontal disease composition shellac dissolved in alcohol and at least one of antibacterial constituent such as an anti-bacteria antibody. EP 1 679 078 A describes the use of natural resin compositions for the treatment of diabetic wound healing and gangrenous wounds. EP 1 399 128 A discloses colloidal compositions for the treatment of digital dermatitis in cows.

**Summary of the Invention** Any references in the description to methods of treatment refer to the compounds, pharmaceutical compositions and medicaments of the present invention for use in a method for treatment of the human (or animal) body by therapy (or for diagnosis).

Broadly, the present invention provides liquid dressing compositions that are capable of forming a self-supporting barrier when applied topically to a subject, in particular for veterinary use as set out in the claims, for example the prevention and/or treatment of wounds and/or infection, including wounds and/or infected sites or lesions associated with digital dermatitis. The dressing will be anti-infective and thus resist bio-film formation from the environment or even from the wound itself. Advantageously, the barrier may be hydrophobic. Also in some situations, the dressing may release its antimicrobial ions into the wound at concentrations that are compatible with antimicrobial activity in the wound itself. Described are also liquid dressing compositions that may have substantially no microbial repellent activity per se, but help to prevent microbial colonisation of wounds by providing a physical barrier to infection. In general, the liquid dressing compositions comprise shellac and a metal-based active in a volatile solvent, such as ethanol, and are capable of forming a self-supporting solid hydrophobic barrier after being topically applied as a liquid to a subject. The metal-based active component may improve barrier function and additionally provides intrinsic repellent properties towards environmental pathogens. As shellac compositions without the inclusion of a metal based active are soluble under alkaline conditions (pH> 7), it is surprising that the compositions of the present invention are capable of forming barriers that can withstand high pHs, for example as found in alkaline environments, such as faecal slurry.

The metal-based active confers anti-infective properties on the liquid dressing compositions of the present invention. Additionally the metal active may help to improve one or more properties of the liquid dressing compositions, for example improving the barrier properties of the compositions and/or a physicochemical property such as the viscosity of the composition.

Alternatively or additionally, the liquid dressing compositions used in accordance with the methods of the present invention provide adhesive, rapid-setting chemical barriers that are capable of locking in pre-applied treatments to the subject, for example at a site of wounds, lesions or infection. Thus, the composition of the invention may be used in combination with other agents such as antibiotics, anti-infective agents or lesion-healing promoting agents or other materials of potential benefit to the host. The barriers formed by the composition can also protect lesions, such as those resulting from digital dermatitis, from the environment, while providing a temporary barrier that withstands extreme conditions, such as alkaline slurry, but is still capable over time of self-degrading. The barrier formed by the compositions of the present invention may have the further advantage that additional forms of bandaging or dressing are not required to physically support the barrier function or efficacy. In preferred applications, the components of the antimicrobial compositions and the barrier formed from them are food-chain compatible.

Accordingly, the present invention not only aims to enhance animal welfare, helping to ensure optimal milk production and reduce antimicrobial losses into the environment and the food chain, but also find application for prevention and treatment of wounds and/or infections in other situations as set out further below. The compositions of the present invention are also biocompatible and their pH can be controlled such that it is maintained above pH 3, preferably above pH 4, and most preferably above pH 4.5 to enable safe, biologically-compatible topical use.

Accordingly, in one aspect, the present invention provides a liquid dressing composition for use in the treatment or prevention of infection and/or wounds, wherein the composition comprises shellac, an anti-infective metal-based active selected from one or more of Cu, Zn, Fe, Al, Mo and Ca, wherein where the anti-infective metal-based active is based on zinc it is selected from one or more of zinc chloride and zinc acetate or zinc carboxylate complexes, and a volatile solvent, wherein the composition is capable of forming a barrier when topically applied to a subject, wherein the composition is for veterinary administration and topically applied to a subject does not include application to the teeth of the subject..

While these compositions were especially developed to provide a treatment of digital dermatitis, notably that occurring in cows, the compositions and veterinary medical uses described herein have applications in the treatment of non-human animal subjects and for the treatment of other types of wounds and/or infections and topical requirements.

In the veterinary medical uses described herein, the topically deposited barriers produced by the compositions of the present invention may be employed in conjunction with conventional treatments, for example by using the barrier compositions over a site or location on a subject where antibiotics or other anti-infectives, therapeutics to promote healing or other therapeutics have been pre-applied. This means that the barrier provides a protective layer that keeps the pre-applied therapeutics locked in. Indeed, it may be advantageous to have a pre-applied ointment or other substance to the skin or wound that acts to prevent 'solvent sting' of the liquid dressing compositions of the present invention when applied topically.

Generally, the compositions used in the present invention have the advantage that they are easily applied as a liquid or gel, are rapid setting to form into an insoluble, water resistant (hydrophobic) barrier and/or are sufficiently robust to provide resistance to mechanical damage and harsh environmental pressures. These advantages are particularly useful for veterinary applications of the present invention where easily applied, but rugged barriers are required, for example for burns and other situations of trauma, including surgery. The property of the compositions being capable of forming self-supporting barriers, and therefore not requiring use in conjunction with bandages or other dressings, is particularly advantageous for use in rugged environments. This is because the barriers formed by the compositions generally have a degree of physical strength to withstand physical damage (e.g. knocks) and are water resistant and can conveniently be carried and applied as a simple liquid format.

The barrier forming properties of compositions used in the present invention also make them suitable for rapid wound or lesion or blemish coverage. This may be useful in time saving situations such as surgical intervention, or to access hard to reach sites on the body of the subject, helping to avoid the need for repeated bandaging, especially in otherwise difficult-to-bandage anatomical areas, thereby improving compliance in non-human animal subjects. Surgical wounds, diabetic wounds and ulcers, intertrigo or skin fold dermatitis, dermatological conditions, traumatic lesions, abscesses and minor scratches or lesions (e.g. from animal fights) are examples of where simple, but effective, chemical barriers may be preferred to other treatment options.

In a further aspect, the present invention, used for veterinary applications, is similarly particularly useful for surgical wounds, dermatological conditions, ulcers, cuts, abrasions and other traumatic wounds. It may for example be used by vets or farmers or by pet owners or by zoos and similar institutions. The versatility and ease of application enables rapid use and access to difficult areas of the body on lesions, wounds or blemishes that, especially, if left untreated, could develop into an infection. Examples of veterinary conditions that may be treated in accordance with the present invention include folliculitis, impetigo, ringworm, rain scald, foot rot, foot and mouth disease, bluetongue, mange and/or pediculosis.

In a further aspect, compounds with an unpleasant taste may be added to compositions intended for veterinary use to discourage animals from biting and chewing at the barrier site.

Preferably, the compositions used in the present invention also provide barriers that are capable of inhibiting further infection ingress at the site of an existing wound, lesion or blemish or an area where there is such risk. Other types of wounds that might be treated according to the present invention include pustules, blisters, erosions, fissures and maceration.

As used herein the treatment of wounds includes chronic wounds, acute wounds, lesions, and/or blemishes, for example sores, bites, ulcers, rashes, burns, abscesses, perforations, incisions, scratches, tears, bumps, skin cracks, blisters, warts, and/or scabs.

Examples of topical ailments and/or injuries that may be associated with, or lead to wounds, and can thus be treated or prevented by the invention described herein include dermatitis, insect bites, other animal bites, traumatic wounds, post-surgical wounds, self-inflicted wounds, skin cancers, thermal burns, electrical burns, chemical burns, radiation burns, ice burns, pox diseases, macules, papules, pustules, plaques, fistulas, comedones, erosions, sinuses, bullae, scales, scars, hives, scaly skin, pimples, vesicles, petechiae, viral infections, bacterial infections, fungal infections, yeast infections, acne, seborrhoea, mange, insect hypersensitivity, allergic eczema, non-allergic eczema and/or hyperpigmentation.

Further skin ailments and/or injuries that can be treated or where subsequent wounds prevented by the compositions disclosed herein include laceration, avulsion, puncture or abrasion wounds, first degree burns, second degree burns, third degree burns, frostbite, necrolytic dermatitis, cutaneous lupus erythematosus, tick bites, flea bites, scabies, aural plaques, papillomatosis, Buruli ulcers, besnoitiosis, urticaria, myiasis, horn cancer, lumpy skin disease, contagious pustular dermatitis, digital dematitis, photosensitization, sheep pox, goat pox, cow pox, fowl pox, swine pox, sarcoids, worm nodule disease, canine pyoderma, dermatophilosis, folliculitis, impetigo, ringworm, rain scald, foot rot, foot and mouth disease, bluetongue, sheep scab, and/or pediculosis, bovine farcy, feline acne, malassezia dermatitis, carbuncles, boils, eosinophilic granuloma with collagen degeneration, skin inflammation, red leg syndrome, melanomas, non-melanoma skin cancers, sore mouth, skin or gill flukes, scale or gill infections, winter ulcer disease, crustacean parasites, feather plucking, scaly face, leg mites, feather mites, beak and feather disease, feather loss, feather damage, cutaneous neoplasms, lice, flies, gnats, ticks, bumblefoot, split keel, wing tip oedema, ichthyosis, myiasis, shell fracture, dysecdysis, scale rot, blister disease, erythema, petechiae, ecchymoses, leeches, gum disease, diabetic ulcers, acral lick dermatitis, pemphigus or skin.

Lesions that may be treated or prevented by the present invention may be of varying appearance and configuration, for instance linear, annular, nummular, target, serpiginous, herpetiform and/or zosteriform

In some instances, superficial skin conditions may, when untreated, lead to the development of more severe wounds, for instance rashes may result in self-inflicted skin perforations due to scratching, biting, pecking, rubbing or other physical actions by the subject. Accordingly, the present invention may be used in such superficial skin conditions.

In some instances, compositions in the present invention may aid the maintenance of a healthy skin and thus prevent lesions in veterinary subjects. Accordingly, examples of skincare actions by compositions of the present invention include, but are not limited to, cleansing, hydrating, moisturising, refining, smoothing, exfoliating, softening, restoring, unclogging, sunscreening, sun protection, heat protection, moisture protection, absorbing, de-sensitising, rejuvenating, clearing impurities, reduce skin irritation, soothing, refreshing, fur maintenance and/or sanitizing.

For the avoidance of doubt, skincare, treatment or prevention of infections and/or wounds include, but are not limited to, preventative or corrective measures or treatments in skin, mucous membrane, hair, fur, nail, bone, claw, hoof, shell, scale, horn, feather, tusks, antlers or teeth in living veterinary subjects.

Preferably, the compositions of the present invention are made from safe (e.g. GRAS-compliant) bio-compatible components, for example components that are already present in the food chain. For veterinary uses, this is important for farm animal-derived food (milk, cheese, meat etc.) entering the human food chain. This also means that the barrier produced using the compositions of the present invention may be left to gradually self-degrade, avoiding the need for a veterinarian or farmer to remove it at the end of a treatment protocol. There is also an inherency in safety of application and a greater appetite for use by users.

In some uses, the compositions of the present invention are coloured, either by virtue of the components from which they are made or by including a dye or other pigment in the composition. Advantageously, this may be used to allow colour tracking when the compositions are applied (e.g. so that the extent of the application to the body of the subject can be determined) and/or to allow the bio-degradation of the barrier to be followed, for example to determine when re-application of the composition is required. Examples of dyes that may be used in accordance with the present invention include synthetic dyes, such as Patent Blue V, or Brilliant Blue FCF, or Brilliant Black BN, or Sunset yellow FCF, or Quinoline Yellow, or Ponceau 4R, or Indigo carmine, naturally derived dyes such as lutein, or riboflavin, or caramel, or chlorophyllin, or carotenes, or betanin, or anthocyanins, and/or mineral dyes such as titanium oxide, or iron oxides and hydroxides.

As demonstrated in the examples, the raw materials used to produce the compositions of the present invention are relatively inexpensive and compositions do not entail complex synthesis as manufacturing is carried out by dissolving and mixing of the various components in ethanol or similar solvent, followed by packaging, ready for use.

In use, treatment using the compositions of the present invention will typically entail optionally applying a pre-administered therapeutic such as an antibiotic compound to a treatment site on the subject and thereafter applying the barrier compositions to that same site so that a hydrophobic barrier is formed through evaporation of the volatile solvent. In the case of the treatment of bovine digital dermatitis, or indeed for other situations, the method may include the initial step of washing the treatment site (i.e. the hoof or leg of the animal with digital dermatitis).

Embodiments of the present invention will now be described by way of example and not limitation with reference to the accompanying figures. However, various further aspects and embodiments of the present invention will be apparent to those skilled in the art in view of the present disclosure.
"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

### Brief Description of the Figures

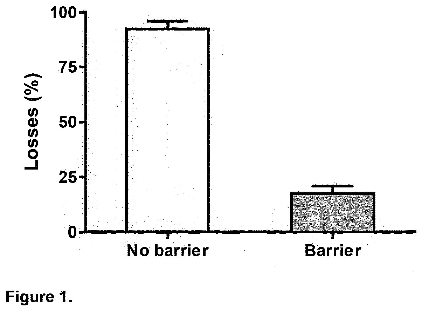

**Figure 1****.** *Ex vivo* losses of antibiotic spray to simulated slurry in the presence or absence of a protective barrier.
**Figure 2****.** Metal release from barriers (Zn and Cu). Samples were collected directly from the contact killing assay after 24h in LB (bacterial medium) at 30°C under mild agitation. The top graph refers to absolute concentrations (ppm) whereas the bottom graph refers to relative release.
**Figure 3****.** Contact killing results. Log CFU/ml after 24h incubation with *E*. *coli* K12 in LB. F0, which consists of a shellac-only composition, had the same bacterial growth as control experiment (no shellac). Note: 1.7 Log CFU/ml is the limit of detection and therefore for the materials that had this result (F4, F6, F8, F11, F13, F14, F26, F27 and F30, see Table 7) no bacterial colonies were formed.
**Figure 4****.** *In vivo* improvement in mobility scores between day 0 and at 7 days posttreatment (n = 7). Statistical analysis was via single tail Wilcoxon matched-paired test.

### Detailed Description of the Invention

### Compositions of the present invention

In the compositions of the present invention, shellac or shellac derivatives may be used and references herein to "shellac" includes both shellac and shellac derivatives. This may include wax-free shellac, dewaxed shellac, dewaxed bleached Shellac, dewaxed and decolourised shellac, shellac ester or waxed shellac. Additionally, the shellac used may be a powder, flakes, lumps or, in certain cases, can be supplied as a solution. Shellac is a resin secreted by the female lac bug on trees and is generally produced in India and Thailand. It is processed and is generally sold as dry flakes or it can be dissolved in solvents such as alcohol to make liquid shellac. Shellac is readily commercially available from suppliers such as Sigma or AF Suter & Co. Preferably, the barrier compositions of the present invention comprise 10% w/w to 70% w/w shellac, more preferably 20% w/w to 70% w/w shellac, more preferably 30% to 70% shellac, more preferably 35% to 70% shellac, more preferably 35% to 60% shellac, and most preferably 40% to 60% shellac, where the amount of shellac refers to the % w/w amount in the compositions before they are applied. In some embodiments, it is preferred that the type of shellac used is one that is approved as a food additive.

Advantageously, the pH of the compositions of the invention can be controlled, for example by using a component with buffering properties. This is generally done so that the compositions have a pH suitable for topical application to a non-human animal subject. A pH above pH 3, preferably above pH 4, and most preferably above pH 4.5 is suitable for safe, biologically-compatible topical use. As an upper limit of pH, preferably the composition used in accordance with the present invention has a pH below 9.0, more preferably below pH 8.5, more preferably below pH 8.0, more preferably below pH 7.4, and most preferably below pH 6.5.

The present inventors found that the use of shellac or shellac derivatives is preferable to use of other resins, some of which have undesirable properties, such as rosin having allergy concerns.

Generally, the composition used in the present invention may be obtained by mixing the components in the volatile solvent to produce a homogenous mixture. It is usually advantageous to dissolve the metal active in the volatile solvent, followed by any additives and finally to add the shellac. This sequence allows metal compounds to dissolve properly prior to the addition of additives (such as PEG, TEC and glycerol) and helps to avoid the resultant mixture being heterogeneous, e.g. due to lumps of shellac. The sequence of steps also means that the additives are well dispersed before adding the shellac as some additives can cause shellac to precipitate when at higher concentrations prior to dispersal in the composition. In some cases, the metal active will be an anti-infective metal active.

In most cases, where additives were added to the compositions described herein, values were recorded on a mass basis. Consequently, concentrations may be referred to as mMolal (mmol /kg) rather than mM (mmol/L). Additionally, where % is used it refers to mass w/w %.

In the present invention, the hydrophobic barriers produced using the compositions of the present invention are "self-supporting", i.e. they are mechanically robust and do not require stabilisation with a structural matrix, for example a woven or non-woven matrix, such as a bandage or gauze as used in the prior art. However, in some cases it may be desirable to provide barriers with increased mechanical strength and, in these situations, the barrier composition may further comprise fibres. Examples of fibre components include, but are not limited to, cellulose, preferably between 1.0 and 15 wt% and more preferably between 2.5 and 5.0 wt%.

Preferably, the volatile solvent used to produce the compositions of the present invention is ethanol, acetone-water mixtures, isopropanol, propanol or butanol, and combinations thereof. The use of ethanol, acetone-water mixtures, propanol and butanol and isopropanol may be preferred for biocompatibility reasons. In cases where an alcohol is used as the solvent, it may be a primary, secondary or tertiary alcohols.

Examples of other volatile solvents (e.g. those with boiling points below 100°C) that may be employed in the present invention include ethers such as diethyl ether, or tetrahydrofuran, ketones such as methyl ethyl ketone, primary alcohols such as methyl alcohol, or ethyl alcohol, or benzyl alcohol, secondary alcohols such as isopropanol, tertiary alcohols such as tert-butyl alcohol, acetone-water mixtures, ethyl acetate-ethanol mixtures, and/or glycol mixtures with any of the above mentioned solvents. Other possible solvents include aldehydes such as benzaldehyde, carboxylic acids such as acetic acid, or lactic acid, or propionic acid, ethers such as diethylene glycol butyl ether, or diethylene glycol ethyl ether, or diethylene glycol methyl ether, or ethylene glycol butyl ether, or ethylene glycol ethyl ether, or ethylene glycol methyl ether, or dioxane, ketones such as cyclohexanone, primary alcohols such as amyl alcohol, secondary alcohols such as sec-butyl alcohol, or iso-butyl carbinol, tertiary alcohols such as diacetone alcohol, terpenoids such as citronellol, and/or glycol mixtures with any of the above.

The metal active is an anti-infective metal active, while in other cases the metal active may be added for other purposes, for example to modulate the viscosity of the composition. The degree of anti-infective properties imparted by the metal active may also depend on the concentration of the metal active in the composition. Anti-infective elements may have any chemical speciation and include combinations thereof, for example Cu, Zn, Fe, Al, Mo and Ca, and most preferably, Cu, Zn, Mg, Ca, and/or Fe, wherein where the anti-infective metal-based active is based on zinc it is selected from one or more of zinc chloride and zinc acetate or zinc carboxylate complexes.

Examples of the anti-infective metal active used to produce the anti-infective compositions of the present invention comprises a copper, zinc, silver, bismuth and/or cobalt compound and may include selenium. Metal actives include copper carboxylate complexes or zinc carboxylate complexes. Examples of suitable anti-infective metal active compounds are copper chloride, copper acetate, copper oxide, copper oxo-hydroxide, copper hydroxide, zinc chloride, zinc acetate, iron chloride, aluminium chloride, molybdenum acetate, or kaolin. In some cases, combinations of anti-infective metal actives may be used, for example a combination of copper and zinc, which optionally may further include selenium. In other cases one or more anti-infective based metal active may be combined with one or more metal-based excipient and for which the latter may or may not have some anti-infection properties. As shown in the examples shows anti-infective metals used in these materials may or may not lead to a final anti-infective composition . This will depend upon precise formulation including but not limited to concentrations and choice of the metals as well as their chemical speciation. Where it is not desirable for these compositions to have anti-infective properties other metals may be used to modulate the physical properties of the final composition.

Preferably, the metal active or excipient is present in the composition at a concentration between 5 mMolal and 3.5 Molal, more preferably between 25 mMolal and 3.5 Molal, more preferably between 50 mMolal and 3.5 Molal, more preferably between 75 mMolal and 3.5 Molal, more preferably between 100 mMolal and 3.5 Molal, more preferably between 100 mMolal and 2 Molal, more preferably between 100 mMolal and 1.5 Molal, more preferably between 100 mMolal and 1 Molal, more preferably between 100 mMolal and 750 mMolal, more preferably between 100 mMolal and 500 mMolal, more preferably between 150 mMolal and 500 mMolal, most preferably between 150 and 350 mMolal. In some cases, the metal active or anti-infective metal active has the advantage of increasing the viscosity of the composition and/or the thickness of the barrier over a corresponding composition omitting the metal active, i.e. one comprising shellac alone.

Specific examples of copper metal actives include copper EDTA, copper tartrate, copper citrate, copper adipate, copper benzoate, copper maleate, copper malonate, copper gluconate, copper aspirinate, copper citraconate, copper fumarate, copper glutarate, copper clioquinol, copper salicylaldehyde benzoylhydrazone, copper picolinate, copper alanine, copper arginine, copper aspartate, copper gluconate, copper glycinate, copper histidine, copper isoleucine, copper leucine, copper lysine, copper methionine, copper phenylalanine, copper proline, copper serine, copper threonine, copper tyrosine, copper valine, copper formate, copper propionate, copper isovalerate, copper pivalate, copper phenylacetate, copper cyanoacetate, copper chloroacetate, copper hydroxyacetate, copper 2-hydroxypropanoate, copper 2-hydroxybutanoate, copper quinate, copper pyruvate, copper ethoxyacetate, copper sulfate, copper thiocyanate, copper sulfide, copper bromide, copper fluoride, copper nitrate, copper carbonate, copper carbonate hydroxide, copper selenite, copper peroxide, tetraamminecopper sulfate, copper phosphate, or elemental copper.

Generally, it will be known to those skilled in the art that milling otherwise insoluble metal actives can aid formulation of homogenous materials.

The compositions used in accordance with the present invention were found to have viscosities suitable for topical application to a subject, going on to form the protective hydrophobic barrier. Examples of suitable viscosities include greater than 50 cP, more preferably greater than 75 cP, more preferably greater than 100 cP, more preferably greater than 200, more preferably greater than 500, more preferably greater than 750, more preferably greater than 1000 cP, more preferably greater than 2500 cP, more preferably greater than 5000 cP, most preferably greater than 10000 cP. In general, for compositions applied as sprays, the viscosity may be between 10 cP to 3000 cP. In cases where the composition is applied as a paint, more viscous compositions may be used for example having viscosities greater than 1000 cP, and more preferably greater that 2000 cP. Viscosity measurements may be made at room temperature, i.e. at 25°C. In some embodiments, the addition of a metal active serves to increase the viscosity of the composition, for example by at least 50%, more preferably by at least 100%, even more preferably by at least 200%, and most preferably by at least 500%, as compared to a corresponding composition that does not include the metal active as measured at 25°C.

In use, the compositions of the present invention may be used in combination with or co-administered with other therapeutic or non-therapeutic agents, such as one or more antibiotics and/or therapeutics. In some examples, the further active agent may be an antibiotic agent, an antimicrobial agent (e.g. oxytetracycline or chlorhexidine) and/or a haemostatic active. In some embodiments, the compositions may be applied over a site where one or more antibiotics or other anti-infective or therapeutic, disinfectant or other agent have been applied, thereby providing a protective barrier over the site of treatment with the therapeutics helping to prevent loss of the therapeutic, for example by washing off with environmental contact. Alternatively or additionally, the compositions of the present invention may be formulated so that they comprise one or more antibiotics. Examples of antibiotic compound approved for animal use include semisynthetic modifications of various natural compounds, such as the beta-lactam antibiotics, which include the penicillins (produced by fungi in the genus *Penicillium*)*,* the cephalosporins, and the carbapenems. Antibiotics that are still isolated from living organisms include the aminoglycosides, whereas other antibiotics, for example the sulfonamides, the quinolones, and the oxazolidinones, are produced solely by chemical synthesis. Many antibacterial compounds are classified on the basis of chemical/biosynthetic origin into natural, semi-synthetic, and synthetic. Ansamycins are a class of antibiotics, including rifamycin, rifampin, rifampicin, rifabutin, rifapentine, rifalazil, ABI-1657, and analogs thereof, that inhibit bacterial RNA polymerase and have exceptional potency against gram-positive and selective gram-negative bacteria. Beta lactams, such as penicillins (e.g. penzylpenicillin, amoxicillin), carbapenems (e.g. meropenem, imipenem), cephalosporins (e.g. cephalexin, cefuroxime). Naturally occurring aminoglycosides such as streptomycin, neomycin, tobramycin, gentamicin, or semi-synthetic derivatives such as amikacin, netilmicin. Fluoroquinolones (e.g. ciprofloxacin, norfloxacin), macrolides (e.g. erythromycin, clarithromycin), ketolides (e.g. telithromycin). Tetracyclines such as tetracycline, oxytetracycline, chlortetracycline, or doxycycline. Glycopeptides (e.g. vancomycin, teicoplanin), lincosamides (e.g. lincomycin, pirlimycin), amphenicols (e.g. thiamphenicol, chloramphenicol), streptogramins (e.g. pristinamycin), oxazolidinones (e.g. linezolid, cycloserine), pleuromutilins (e.g. tiamulin, retapamulin). Mupirocin, fusidic acid, polymyxin B or Bacitracin are often used topically and may also be used in combination with the invention described herein. Preferred antibiotics, for example those employed topically for hoof and superficial wound infections (such as digital dermatitis), include chlortetracycline, oxytetracycline and thiamphenicol.

In some instances, the compositions described here in can be used in combination with metal-based therapeutics such as copper (II) sulphate, copper EDTA, zinc EDTA, zinc pyrithione, silver, silver nitrate, silver sulfadiazine and mixtures of these metal-based therapeutics. In this situation, the compositions for use in the present invention may be applied after application of the metal-based therapeutic.

In some instances, topical antibacterials (non-antibiotic) may be used. For example, parabens, biguanides such as chlorhexidine, diamines, hydrogen peroxide, phenols such as triclosan, halophenols such as chloroxylenol, iodine, or benzoates such as benzoic acid.

In some instances, anti-fungal agents may also be used. Examples of anti-fungal agents include polyenes (e.g. amphotericin B, nystatin), azoles such as fluconazole, ketoconazole, clotrimazole, miconazole, terconazole, butoconazole, clotrimazole, econazole, oxiconazole, tioconazole, or sulconazole, allylamines and related compounds, such as terbinafine, tolnaftate, butenafine, or naftifine, morpholine-related compounds such as amorolfine, also ciclopirox, gentian violet, selenium sulphide, zinc pyrithione undecylenate and piroctone olamine.

In some instances, antiprotozoals may also be used. For example, metronidazole (also an antibiotic) or nitazoxanide (also active as an antiviral).

In some instances, combinations of the antibiotics or other therapeutics above can also be used to increase efficacy, e.g. neomycin, polymyxin and bacitracin.

In some instances, preferably the antimicrobial composition further comprises one or more hydrophilic additives which are used as a plasticisers, or to accelerate barrier formation, or both, after application of the composition to a treatment site on a subject or individual. Examples of hydrophilic additives include TEC ( wherein the compositions preferably comprise 2 to 30%, more preferably 3 to 20%, most preferably 5 to 15%), polyalkylene glycols such as PEGs including PEG-400 (wherein the compositions preferably comprise 2 to 30%, more preferably 3 to 20%, most preferably 5 to 15% ), glycerol (wherein the compositions preferably comprise 1 to 20%, more preferably 2 to 15%, most preferably 3 to 10% ), hexylene glycol (wherein the compositions preferably comprise 1 to 20%, more preferably 2 to 15%, most preferably 3 to 10%) and triacetin (wherein the compositions preferably comprise 1 to 20%, more preferably 2 to 15%, most preferably 3 to 10%).

The present inventors observed that, in some instances, non-solvents for shellac (i.e. solutions in which shellac is insoluble) may be mixed at the point of application with a shellac-containing solution to induce immediate formation of a solid barrier. For example, a barrier is instantly formed when an ethanolic solution of shellac comes into contact with a sufficient quantity of glycerol. Also, *in situ* mixing can be achieved using a dual component cartridge where one chamber carries a shellac solution and the other a non-solvent (see example 2.1), such as PEG or glycerol. The inventors also observed that this process requires higher quantities of non-solvents compared to their use as hydrophilic additives described above. For example, upon mixing the two components, the resulting concentration of PEG should be 30 to 75%, most preferably 33 to 66%. As an additional example, the resulting concentration of glycerol should be 20 to 75%, most preferably 25 to 50%.

In some instances, preferably the anti-infective composition further comprises one or more hydrophobic barrier enhancing agents, for example to enhance water resistance of the barrier layers formed when the anti-infective composition is applied to a subject or individual and at least a proportion of the volatile solvent has evaporated. Examples of hydrophobic barrier enhancing agents include decanoic acid (wherein the compositions preferably comprise 2 to 30%, more preferably 3 to 20%, most preferably 4 to 10%), octanoic acid (wherein the compositions preferably comprise 2 to 20%, more preferably 3 to 15%, most preferably 5 to 12 %), oleic acid (wherein the compositions preferably comprise 2 to 20%, more preferably 3 to 15%, most preferably 5 to 12 %),) or lauric acid ( wherein the compositions preferably comprise 2 to 20%, more preferably 3 to 15%, most preferably 5 to 12 %).

In some instances, preferably the compositions further comprise one or more buffering agents to inhibit barrier degradation that may otherwise be too rapid. This is particularly advantageous in digital dermatitis, since barriers in contact with alkaline faecal slurries degrade faster and by including an additional buffering agent barrier degradation can be slowed down. This is because shellac may become water soluble under alkaline conditions, and faecal slurry has a pH of about pH 8 and has a buffering capacity of about 100 mM which will contribute to barrier degradation over time due to solubilisation of the shellac. For this reason, it is desirable that the compositions used in accordance with the present invention have a pH that is above 4.0 for it to be suitable topically, but which can be maintained in use below pH 6.0 or 6.5 for as long as possible to avoid such losses. One elegant solution to this problem is to include a buffer in the composition, and preferably a buffer with a pKa between 4.0 and 6.5. Examples of suitable buffer components include carboxylic acids including monocarboxylic acids, such as benzoic acid, lauric acid. glycolic acid, levinulic acid, lactic acid, pyruvic acid, or glyceric acid, di-carboxylic acids, such as adipic acid (preferably 0.5% to 5%), azelaic acid (preferably 0.5% to 5%), succinic acid, pimelic acid or decanoic acid, or a tri-carboxylic acid such as citric acid. It will be known to those skilled in the art that diverse categories of molecule, including but not limited to amino acids, fatty acids, and alpha hydroxy acids, may contain carboxylate pendant groups. Such molecules may also be suitable as buffering agents in the present invention.

In some instances, preferably the composition further comprises an anti-inflammatory agent or a complexing agent to enhance loading or solubility of the metal active. Examples of suitable anti-inflammatory agents or complexing agents are salicylic acid, sodium salicylate and propionic acid, or chemical derivatives thereof.

In some instances, preferably the composition further comprises haemostatic agents. For example, kaolin, aluminium sulphate, or a zeolite may be included in applications where the wound treatment includes blood clotting, such as in the treatment of acute wounds, e.g. in surgical wounds or injuries sustained during sports such as horse riding or by working animals such as farm dogs. Examples of suitable concentrations of a haemostatic agents, such as kaolin, include 1% to 40%, more preferably 2% to 35%, more preferably 3 to 30%, most preferably 5 to 30%.

In some instances, the composition further comprises a painkiller such as acetylsalicylic acid, salicylic acid, trolamine salicylate, magnesium salicylate and/or methyl nicotinate. Natural painkillers, such as capsaicin, may also be added.

In some instances, the composition further comprises an anti-inflammatory agent, such as ibuprofen, diclofenac, felbinac, ketoprofen and/or piroxicam.

In some instances, the composition further comprises a soothing agent, such as menthol, eucalyptus oil, cinnamon oil and/ or camphor.

In some instances, the composition further comprises an anti-itching agent such as hydrocortisone.

Preferably, all components of the compositions are approved food or feed additives and/or qualify as GRAS reagents ("Generally Recognised As Safe").

### Formulations and Uses

The compositions for use in accordance with the present invention are employed for the prevention or treatment of infection and/or wounds. Where they are used for the treatment or prevention of infection the infection may be a bacterial infection, a fungal infection, a parasitic infection or a viral infection. In general, the compositions are particularly suitable for the treatment or prevention of bacterial infection, that is the compositions are antimicrobial compositions. Generally, the compositions used in accordance with the present invention comprising shellac and an anti-infective metal active in a volatile solvent are capable of forming a hydrophobic barrier when topically applied to a non-human animal (e.g. mammalian) subject. On topical application to the subject, the compositions of the present invention generally form a solid interface such that a barrier is formed within a few minutes (e.g. within 1, 2, 3, 5 or 10 minutes). However, this is not essential since contact with environmental moisture, or in the case of the treatment of farm animals, such as cows, the faecal slurry, will result in immediate formation of a hydrophobic barrier, making the present invention well adapted for use in challenging environments, such as digital dermatitis (e.g. bovine digital dermatitis) and udder infection, for example udder ulceration, udder dermatitis or udder lesions. Further veterinary uses include the use of the liquid dressing compositions for teat sealing or horn removal.

The anti-infective compositions used in the veterinary uses according to the present invention can be used in general wound management especially where infections are a potential burden. These include chronic ulcers and acute wounds including traumatic wounds or post surgical wounds. Examples of wounds where the present compositions are useful include open wounds (laceration, avulsion, puncture or abrasion wounds), or chronic ulcers, such as diabetic ulcers, Buruli ulcers or other types of wounds, such as burns, surgical wounds or for aiding in the treatment of foot rot, traumatic lesions, abscesses and minor scratches or lesions. The compositions used in accordance with the present invention are also advantageous in aiding the treatment of closed wounds that are already underdoing healing by providing a protective environment and preventing re-infection. The compositions may therefore be used in the prevention of wounds. In some embodiments, the liquid dressing compositions will be supplied in the form of an over-the-counter (OTC) composition. Other veterinary conditions treatable in accordance with the present invention include folliculitis, impetigo, ringworm, rain scald, foot rot, foot and mouth disease, bluetongue, mange and/or pediculosis.

The anti-infective compositions according to the present invention are well suited for veterinary applications where wound management of animals can be particularly challenging. These challenges include the high microbial load (pathogenic and non-pathogenic) in the surrounding environment and the fact that frequent wound cleaning and re-application of antimicrobials is generally not feasible or economic. The use of standard dressings is generally discouraged as these accumulate faecal matter or other detritus. In contrast to the use of dressing which requires the isolation of animals for application and removal of the dressing, the compositions used in the present invention can be used to form robust self supporting hydrophobic barriers that are robust enough to protect the wound, and optionally to also lock in the pre-applied antibiotic or antimicrobial, but which degrade over time. This reduces the need for frequent re-application of antibiotics or antimicrobials and makes subsequent removal of the barrier unnecessary. The present invention may also allow reduced doses of antibiotics to be employed. Generally, the hydrophobic barriers formed from compositions of the present invention require 1 to 20 days to degrade, more preferably 2 to 10 days, most preferably between 3 and 7 days. Degradation rates are usually higher when the barriers are in contact with faecal slurry.

In some cases, the compositions used in the present invention do not substantially include silicon dioxide (silica), i.e. the compositions.

Alternatively or additionally, the compositions used in the present invention do not substantially include a flexibilizer, such as an oil, e.g. a non-drying or semi-drying oil. Examples of flexibilizers are triglycerides oils, for example of animal, vegetable, mineral or synthetic origin. Examples of suitable non-drying or semidrying oils include pine oil, eucalyptus oil, tea-tree oil, rosehip oil, soya bean oil, coconut oil, castor oil, olive oil safflower oil and sunflower.

In the present application, by "substantially does not include", it is meant that the compositions of the present invention do not include the stated component, or else only includes a minimal or trace amount of the component in question, for example at less than 1.0% w/w, more preferably less than 0.5% w/w, still more preferably less than 0.1% w/w.

In some cases, the compositions of the present invention consist of or consist essentially of the stated components.

One advantage of the compositions of the present invention is that the barriers are nonporous to water. In cases where the compositions form barrier with pores, they are generally pores of small size, for example having mean diameters which are 1 µm or less in diameter.

The compositions used in the present invention can be applied using any device capable of delivering viscous liquids. Generally the viscosity of the compositions means that they are non-sprayable. Spray-on dressings in the prior art suffer from disadvantages, as they may form relatively thin films since the aerosol used to deposit the film cannot carry a high percentage of solids, have a high ratio of drying time: mass of solids deposited and are generally non-biodegradable. Accordingly, it is preferred that the compositions of the present invention are administered by squeezable bottles, squeezable or collapsible tubes, adhesive dispensers, cartridge and plunger kits using manual, pneumatic or electric cartridge guns, or liquid dispensers. In some instances, it may be preferred that the compositions are administered through the use of disposable spatulas or brushes or similar. In some cases, application may be combined with distributing the composition over the location to be treated, for example by using a brush or other applicator.

In other cases, the compositions of the present invention may be formulated for spray delivery. This may be useful in circumstances in which thinner dressings are appropriate for aiding in the treatment of wounds or managing infection.

Accordingly, in a further aspect, the present invention provides a device for applying a liquid dressing composition for use in the treatment of infection and/or wounds, the device comprising:
a first chamber containing a composition comprising shellac and an anti-infective metal-based active selected from one or more of Cu, Zn, Fe, Al, Mo and Ca in a volatile solvent for use as defined in any one of claims 1 to 20 and a second chamber containing a non-solvent;
a mixing chamber in communication with the first and second chambers;
actuating means for delivering the composition and non-solvent to the mixing chamber to provide a mixed composition, wherein the non-solvent is glycerol or a polyalkylene glycol; and
an applicator in communication with the mixing chamber for applying the mixed composition to a location on a subject, wherein the composition is capable of forming a hydrophobic barrier when topically applied to a subject, wherein the composition is for veterinary administration and where topically applied to a subject does not include application to the teeth of the subject.

In these devices, examples of the non-solvent include glycerol or a polyalkylene glycol, such as PEG. These devices may take the form of a double or dual cartridge applicator device. In other embodiments, the present invention provides devices for delivering compositions in the form of sprays, for example using a pressurised gas or air delivery.

The liquid dressing compositions of the present invention may be formulated for use as antimicrobial agents or antimicrobial agents, for example for the treatment or prevention of bacterial or microbial infections and/or for the treatment of wounds. Accordingly, the compositions of the present invention may comprise, in addition to the materials described above, a pharmaceutically acceptable excipient, carrier, buffer, stabiliser or other materials well known to those skilled in the art. Such materials should be non-toxic and should not significantly interfere with the efficacy of the solid phase materials for the application in question.

For example, it is known in the art that incorporation of antimicrobials within dressings and their subsequent release is advantageous for the prevention of microbial ingress and growth at wound sites. Usefully/surprisingly the present compositions can maintain a sterile dressing (via contact killing) with minimal release of antimicrobial metals, thereby avoiding side-effects and/or off-target effects. This is distinct from previous compositions in the art wherein resins were employed as delivery matrices for antimicrobials. In another embodiment, the compositions of the present invention may be formulated to release their antimicrobial metal ions into the wound at concentrations that are active in the wound environment.

As used herein, the term "anti-infective" includes the treatment or prevention of infections caused by bacteria, fungus, parasites or viruses.

The term "antimicrobial" as used herein includes the treatment or prevention of infections caused by gram negative and gram positive microorganisms and especially refers to preventing ingress of microorganisms from the environment to the wound or colonisation of the dressing by microorganisms including *Spirochaete sp., Escherichia sp.,* such as *E*. *coli, Staphylococcus sp., such* as *S*. *epidermis, S*. *aureus* and methicillin-resistant staphylococcus aureus ("MRSA"), *Bacillus sp.,* such as *B*. *subtilis or B.anthracis, Pseudomonas sp., such* as *P*. *aeruginosa, Vibrio sp.,* such as *V. fisheri, Streptococcus sp., such* as *S*. *zooepidemicus, S. equi, S*. *suis, S*. *uberis, S*. *pyogenes* and *S*. *pneumoniae, Klebsiella sp. such* as *K pneumoniae, Micrococcus sp., such* as *M. luteus, Clostridium sp.* such as *C*. *difficile* or *C*. *perfringens, Acinetobacter sp.* such as *A. baumannii, Mycobacterium sp.,* such as *M*. *tuberculosis and M. bovis, Salmonella sp,* a *Erysipelothrix* sp. such as *E*. *rhusiopathiae* or *E*. *insidiosa, a Corynebacterium* sp. such as *C minutissimum, a Leptospira* sp. such as *L. interrogans, a Cutibacterium sp.* such as *C*. *acnes,,* a *Campylobacter sp.* such as *C*. *fetus,* a *Klebsiella sp.* such as *K*. *pneumoniae, K. rhinoscleromatis, a Capnocytophaga sp.* such as *C*. *canimorsus, a Vibrio* sp. such as *V. vulnificus, a Pasteurella sp.* such as *P*. *multocida,* a *Aeromonas* sp. such as A. *hydrophila, a Eikenella sp.* such as *E*. *corrodens, a Mycoplasma sp.* such as *M. pneumoniae, a Calymmatobacterium sp.* such as *C*. *granulomatis,* , , *a Listeria sp.* such as *L. monocytogenes, a Treponema* such as *T. carateum or T. vincentii or T. maltophilum or T. lecithinolyticum, a Bartonella sp.* such as *B*. *henselae, a Nocardia sp.* such as *N*. *brasiliensis, a Yersinia sp.* such as *Y. pestis,* a *Serratia sp.* such as *S*. *marcescens,* a *Burkholderia sp.* such as *B*. *pseudomallei, a Actinomyces sp.* such as *A. israelii, a Borrelia sp. such* as *B*. *burgdorferi, a Enterococcus sp. such* as *E*. *faecalis* or fungi including *Candida sp., such* as *C*. *albicans, Malassezia sp* such as *M. furfur and M. pachydermatis a Trycophyton sp.* such as *T. rubrum or T. interdigitale, a Microsporum* sp. such as *M. canis, M. equinum, M. audouinii, M. gypseum, or M. nanum, a Epidermophyton Sp.* such as *E. floccosum, a Fusarium sp such as F.. solani, Rhizopus* microspores, *Rhizomucor sp. such* as *R*. *pusillis, Mucor* sp. such as *M*. *indicus, a Syncephalastrum sp.* such as *S*. *racemosumm, a Cunninghamella sp.* such as *C*. *bertholletiae, a Apophysomyces sp.* such as *A*. *elegans, a Lichtheimia* such as *L*. *corymbifera, a Saksenaea sp.* such as *S*. *erythrospora, Aspergillus sp.* such as *A*. *glaucus, a Blastomyces sp.* such as *B*. *dermatitidis, a Coccidioides sp.* such as *C*. *immitis, a Cryptococcus sp.ush* as C. neoformans, a *Histoplasma sp.* such as *H*. *capsulatum, a Cochliobolus sp* such as *C*. *lunatus, a Cladophialophora sp.* such as *C*. *bantiana, a Exophiala sp.* such as *E*. *jeanselmei, , a Pyrenochaeta sp.* such as *P*. *romeroi, a Pneumocystis sp.* such as *P*. *jirovecii, a Paracoccidioides sp.* such as *P*. *brasiliensis, a Sporothrix sp.* such as *S*. *schenckii, a Talaromyces sp.* such as *T. marneffei,* or parasites such as a *Sarcoptes sp.* such as *S*. *scabiei, Leishmania sp* such as *L. donovani, L. braziliensis, L. panamensis,* Ancylostomatidae such as *Ancylostoma sp.* or *Necator sp., a Strongyloides sp.* such as *S*. *stercoralis, a Onchocerca sp.* such as *O. volvulus,* dipterous larvae such as *Lucilia sericata, Protophormia terraenovae, Cochliomyia hominivorax, Cordylobia anthropophaga,* or *Sarcophaga bercaea, Balamuthia sp* such as *B. mandrillaris,* or *Acanthamoeba sp.,* or a virus such as Papillomavirus, an enterovirus such as Coxsackievirus, a Molluscipoxvirus sp. such as Molluscum contagiosum, a Varicellovirus, or a herpesvirus. The term "antimicrobial" as used herein is understood to apply to substances including those which inhibit microbial attachment to surfaces, kill microbes and/or inhibit microbial reproduction and especially prevent microbial ingress to the host from the environment or colonisation or biofilm formation of the dressing by microbes. The term "microbe" is understood to include all microorganisms, including bacteria as set out above, as well as fungi such as yeast, archaea and protists and viruses. The terms "microbial" and "antimicrobial" should be interpreted accordingly.

In some embodiments, the liquid dressing compositions of the present invention may be used for the treatment or prevention of infection and/or wounds, where the infection is caused by a virus.

Examples of viruses that may infect a subject and which can be treated or prevented using the compositions of the present invention include respiratory viruses, gastrointestinal viruses and skin viruses.

Respiratory viruses: Rhinoviruses, canine adenoviruses (CAV1and CAV2), canine parainfluenza viruses (type 5), bovine parainfluenza virus type 3, Influenza virus (e.g. Influenza virus A - H3N2, H1N1, H2N2, H5N1, H1N2, H9N2, H7N9 ), respiratory syncytial virus (Bovine RSV, pneumonia virus of mice), naturally circulating alpha- and beta-coronaviruses (e.g. Canine CoV, Feline CoV, Bovine CoV), Epstein-Barr virus, cytomegalovirus, type 1 bovine herpesvirus, Varicella-zoster virus.

Gastrointestinal viruses: Rotaviruses (e.g. avian and bovine), bovine adenovirus (), bovine viral diarrhoea virus, caliciviruses and astroviruses.

Skin viruses: canine parvovirus, , Epstein-Barr virus, cytomegolavirus, dengue viruses, zika virus, monkeypox virus, cowpox virus, bovine papillomavirus 1 and 2, foot-and-mouth disease virus.

The groups of viruses include respiratory viruses, gastrointestinal viruses and skin viruses. Accordingly, the liquid dressing composition for use in the treatment or prevention of infection and/or wounds, wherein the infection is caused by a virus in the Adenoviridae family, such as canine adenovirus 1, or by a Caliciviridae virus such as norovirus, or feline calicivirus, or sapovirus, or by a virus in the Coronaviridae family, such as Canine CoV, or Feline CoV, or Bovine CoV, or porcine epidemic diarrhoea virus or by a virus in the Flaviviridae family, such as Dengue virus, or Japanese encephalitis, or yellow fever virus, or hepatitis C virus, or zika virus, or West Nile virus, or pestivirus C or by a virus in the Herpesviridae family, such as Marek's disease virus, Suid herpesvirus-1, equine herpesvirus-1 or varicella-zoster virus, or Epstein-Barr virus, or cytomegalovirus or by a virus in the Orthomyxoviridae family, such as influenza virus A H3N2, or influenza virus A H1N1, or influenza virus A H2N2or by a virus in the Papillomaviridae family, such as bovine papillomavirus type 1, or 2 or by a virus in the Paramyxoviridae family, such as bovine parainfluenza virus type 3, or Avian orthovulavirus 1, or small ruminant morbillivirus virus or by a virus in the Parvoviridae family, such as canine parvovirus, or porcine parvovirus or by a virus in the Poxviridae family, such as lumpy skin disease virus, sheeppox virus, goatpox virus, Monkeypox virus, or cowpox virus or by a Reoviridae virus such as bluetongue virus, African horse sickness virus, rotavirus A or rotavirus B, or rotavirus C, or by a virus in the Phenuiviridae family, such as Rift Valley fever virus .

The present invention has application in the veterinary field, for example for use in the treatment of a non-human animal, and more especially non-human mammals, for example companion animals such as dogs, cats and horses, as well as livestock species, such as cows, goats and sheep. Rarer / minor species (e.g. M.U.M.S., minor use minor species) including those used in farming such as alpacas or llamas, exotic pets, zoo animals and those in safari parks and similar are also potential beneficiaries of these new materials. In one particular application, antimicrobial compositions of the present invention are used for the treatment of bovine digital dermatitis, and more particularly for the treatment of dairy cows. In further applications, antimicrobial compositions of the present invention are used for udder dermatitis and post amputation, including claw amputations, in cows; foot rot in sheep; mud fever in horses. Composition of the present invention can also be used in post surgical protection of wounds and stitches including neutering procedures in dogs, cats and horses as well as sheep, pigs and cows. Examples of species treatable using the present invention include cows, buffalos, yak, pigs, horses, sheep, goats, llamas, alpacas, deer, donkeys, zebus, zebras, elephants, orangutans, chimpanzees, gorillas, lemurs, gibbons, baboons, chickens, turkeys, ducks, emus, geese, ostrich, cats, dogs, ferrets, gerbils, hamsters, chinchillas, rats, rabbits, reptiles, amphibians, parrots, canaries, galliformes, anseriformes and passeriformes.

For some veterinary applications in which an animal might chew, bite or peck the barrier formed by the compositions of the present invention, it may be desirable to include a taste deterrent, e.g. a bitter component, to the composition to discourage such behaviour and to extend the life of the barrier or remove the need for re-application.

In embodiments in which the compositions are intended for the administration to a subject, for example in the treatment of wounds or skin infections, the precise nature of the carrier or other component may be related to the manner or route of administration of the composition, typically via a topical route.

In one embodiment, the compositions of the present invention may be formulated for topical administration useful in the treatment of wounds, ulcers or the treatment or prevention of bacterial infection. The application of the present invention with topical products aids therapeutic use for wound healing.

In some cases, an effective amount of antimicrobial compositions herein may be formulated for topical application to animals to the skin, hide, fur, feathers or wattle. In general, "topical administration" according to the present invention does not include application to the teeth of the subject.

In some cases, for example where the compositions are used for the treatment or prevention of respiratory virus infection, the composition may be administered nasally, for example using a loaded swab that lines the nasal passage or goes on outside of nose.

### Examples

The following examples are provided to illustrate preferred aspects of the invention.

### Example 1: Primary Formulations

### Example 1.1. S1B1

30% shellac de-waxed, 100 mMolal copper acetate, 100 mMolal zinc chloride in ethanol. Copper acetate, and zinc chloride were weighed into a 50 mL falcon tube and then ethanol was added. The mixture was manually homogenised and left mixing for ca. 1 hour. Next shellac was added into the plastic bottle and the mixture was again left mixing until homogenous.

### Example 1.2. S1B2

40% shellac de-waxed, 100 mMolal copper acetate, 100 mMolal zinc chloride in ethanol. As per Example 1.1. except shellac concentration was 40% instead of 30%.

### Example 1.3. S1B3

50% shellac de-waxed, 80 mMolal copper acetate, 20 mMolal copper chloride 100 mMolal zinc chloride in ethanol. Copper acetate, copper chloride and zinc chloride were weighed into a 500ml plastic bottle and then ethanol was added. The mixture was manually homogenised and left mixing for ca. 1 hour. Next shellac was added into the plastic bottle and the mixture was again left mixing until fully homogeneous.

### Example 1.4. S1B4

60% shellac de-waxed, 50 mMolal copper acetate, 50 mMolal copper chloride 100 mMolal zinc chloride in ethanol. Copper acetate, copper chloride and zinc chloride were weighed into a 500ml plastic bottle and then ethanol was added. The mixture was manually homogenised and left mixing for ca. 1 hour. Next shellac was added into the plastic bottle and the mixture was again left mixing until fully homogeneous.

### Example 1.5. S1B5

40% shellac de-waxed (blended), 12% PEG-400, 5% glycerol, 100 mMolal copper acetate, 100 mMolal zinc chloride, in ethanol. As per Example 1.2. except that PEG-400 and glycerol were added to the ethanolic metal solution and mixed well prior to shellac addition.

### Example 1.6. S1B6

40% shellac de-waxed (blended), 8% triethyl citrate (TEC), 1% ZnO, 100 mMolal copper acetate, 100 mMolal zinc chloride, in ethanol. As per Example 1.2 except that TEC and ZnO were added to the ethanolic metal solution and mixed well prior to shellac addition.

### Example 1.7. S1B7

50% Shellac de-waxed (blended), 10% triethyl citrate (TEC), 5% decanoic acid, 100 mMolal copper acetate, 100 mMolal zinc chloride, in ethanol. Formulation as per Example 1.2 except that concentration of shellac was 50% instead of 60% and TEC and decanoic acid were also mixed prior to addition of shellac.

### Example 1.8. S1B8

Formulation as per Example 1.5, but with higher metal concentrations (190 mMolal zinc chloride, 126 mMolal copper acetate) and with 109 mMolal salicylic acid. 1.204g of copper acetate, 1.236g of zinc chloride and 0.7147g salicylic acid were added to 17.38g of ethanol and mixed well. Glycerol (2.1236g), PEG-400 (5.1359g) and shellac (19.75g) were added subsequently and the slurry mixed until homogenous.

### Example 1.9. S1B9

43% shellac, 174 mMolal copper acetate, 174 mMolal zinc chloride, 101 mMolal salicylic acid, 8% glycerol,10.5% PEG 400, 4.3% azelaic acid, 2.2% decanoic acid, 1.8% adipic acid. 1.2093g of copper acetate, 0.8273g of zinc chloride and 0.4843g salicylic acid were added to 7.82g of ethanol and mixed well. Azelaic acid (1.5053g), decanoic acid (0.756g), adipic acid (0.642g), Glycerol (2.802g), PEG-400 (3.6421g) and shellac (15.1195g) were added subsequently and the slurry mixed until homogenous.

### Example 1.10: High metal salt (not according to the invention)

500 mMolal copper chloride, 450 mMolal zinc chloride, 50 mMolal zinc oxide, shellac 40%.

### Example 1.11: Shellac plus metal hydroxides

500 mMolal copper chloride and 500 mMolal zinc chloride (as per final concentration), neutralised to pH 4 with KOH, then shellac added to 40% w/w.

### Example 1.12: Kaolin-shellac formulation

3.92g of kaolin was added to 15.65g of 50% (w/w) esterified shellac in ethanol and mixed well. Kaolin has been used as a source of antimicrobial aluminium and as a haemostatic.

### Example 1.13: S1B5 with kaolin

40% shellac de-waxed (blended), 12% PEG-400, 5% glycerol, 100 mMolal copper acetate, 100 mMolal zinc chloride, in ethanol. Copper acetate, and zinc chloride were weighed into a 50 mL falcon tube and then ethanol was added. The mixture was manually homogenised and left mixing for ca. 1 hour. PEG-400 and glycerol were added to the ethanolic metal solution and mixed well prior to shellac addition. After the shellac was fully dispersed, kaolin was added at 11% (w/w).

### Example 2. Dual Component Formulations

### Example 2.1

The material produced in Example 1.4 was loaded into one chamber of a dual component cartridge, the other chamber containing glycerol, and applied to an active digital dermatitis lesion with a manual applicator gun. Upon mixing of the two components, a gum-like barrier was immediately formed. This demonstrates the feasibility of delivering the compositions of the present invention using applicator gun devices.

### Composition Compilation Table

### Key for sources and types of shellac:

S - Wax-free shellac from Sigma
AFD - De-waxed (<0.5%) shellac from AF Suter & Co
AFW - Waxed shellac from AF Suter & Co
AFE - Pre-esterified shellac (Shellac Ester) from AF Suter & Co
F - for shellac used as obtained from supplier, i.e. flakes (as obtained from supplier)
B - for shellac used after blending the flakes into powders

### Key for sources of metals:

CuAc - Cupric acetate hydrate (Cu(CH₃COO)₂.xH₂O)
FeCl - Ferric chloride hexahydrate (FeCl₃.6H₂O)
CuCl - Cupric chloride (CuCl₂.2H₂O)
ZnCl - Zinc chloride (ZnCl₂)
Zn Ac - Zinc acetate dihydrate (Zn(CH₃COO)₂.2H₂O)
AICI - Aluminium chloride (AlCl₃.6H₂O)
MoAc - Molybdenum acetate (Mo₂(CH₃COO)₄)

**Table 1. Examples of compositions comprising shellac and antimicrobial metals. Unless stated otherwise, ethanol was used as bulk solvent.**

| **Shellac** | **Metal 1** | **Metal 2** | **Metal 3** | **Additives** |
|---|---|---|---|---|
| **25% AFD (B)** | 150 mMolal CuAc | | | |
| **29% AFD (B)** | 488 mMolal FeCl | | | |
| **10% S (F)** | 100 mMolal CuCl | 100 mMolal ZnCl | | |
| **25% AFD (B)** | 150 mMolal CuAc | | | |
| **29% AFD (B)** | 488 mMolal FeCl | | | |
| **30% AFD (B)** | 100 mMolal CuCl | | | 63% acetone, 5% UHP ^{a} |
| **30% AFD (B)** | 792 mMolal FeCl | | | |
| **30% AFD (B)** | 399 mMolal CuCl | | | |
| **30% AFD (B)** | 150 mMolal CuAc | | | 3% Triethyl citrate (TEC) |
| **30% AFD (B)** | 150 mMolal CuAc | | | |
| **30% AFD (B)** | 100 mMolal CuAc | 100 mMolal ZnCl | | |
| **30% AFD (B)** | 100 mMolal CuAc | 100 mMolal ZnCl | | 5% Oleic acid |
| **30% AFD (B)** | 50 mMolal CuAc | 100 mMolal ZnCl | | |
| **30% AFD (B)** | 100 mMolal CuCl | 100 mMolal ZnCl | | |
| **30% AFD (B)** | 50 mMolal CuAc | 100 mMolal ZnCl | | Lauric acid 5% |
| **30% AFD (F)** | 100 mMolal CuAc | 100 mMolal ZnCl | 1% ZnO | |
| **30% AFD (F)** | 100 mMolal CuAc | 100 mMolal ZnCl | 2% ZnO | |
| **30% AFD (F)** | 100 mMolal CuAc | 100 mMolal ZnCl | | |
| **30% AFD (F)** | 100 mMolal ZnCl | | | |
| **30% AFD (F)** | 100 mMolal CuAc | | | |
| **30% AFD (F)** | 100 mMolal CuAc | 100 mMolal ZnCl | | 5% glycerol |
| **30% AFD (F)** | 100 mMolal CuAc | 100 mMolal ZnCl | | 10% glycerol |
| **30% AFD (F)** | 100 mMolal CuAc | 100 mMolal ZnCl | | 5% Triacetin |
| **30% AFD (F)** | 100 mMolal CuAc | 100 mMolal ZnCl | | 10% Triacetin |
| **30% AFD (F)** | 100 mMolal CuAc | 5% ZnO | | |
| **30% AFD (F)** | 100 mMolal CuAc | 100 mMolal ZnCl | | 5% Decanoic acid |
| **30% AFW (F)** | 100 mMolal CuAc | 100 mMolal ZnCl | | |
| **30% S (F)** | 100 mMolal CuCl | 100 mMolal ZnCl | | |
| **30% S (F)** | 100 mMolal CuAc | 100 mMolal ZnCl | 5% ZnO | |
| **30% S (F)** | 100 mMolal CuAc | 100 mMolal ZnCl | 15% ZnO | |
| **30% S (F)** | 100 mMolal CuAc | 100 mMolal ZnCl | | 5% glycerol |
| **30% S (F)** | 50 mMolal CuAc | 50 mMolal CuCl | 100 mMolal ZnCl | 5% glycerol |
| **30% S (F)** | 100 mMolal CuAc | 10% ZnO | | |
| **30% S (F)** | 100 mMolal CuAc | 10% ZnO | | 5% glycerol |
| **30% S (F)** | 50 mMolal CuAc | 50 mMolal CuCl | 10% ZnO | |
| **30% S (F)** | 100 mMolal CuAc | 100 mMolal ZnCl | | 10% Decanoic acid |
| **30% S (F)** | 50 mMolal CuAc | 50 mMolal CuCl | 10% ZnO | 5% glycerol |
| **35% AFD (F)** | mMolal CuAc | 100 mMolal ZnCl | | |
| **35% AFD (F)** | 100 mMolal CuAc | 100 mMolal ZnCl | 5% ZnO | |
| **35% AFD (F)** | 50 mMolal CuAc | 50 mMolal CuCl | 100 mMolal ZnCl | |
| **35% AFD (F)** | 50 mMolal CuAc | 50 mMolal CuCl | 5% ZnO | |
| **35% AFD (F)** | 100 mMolal CuAc | 100 mMolal ZnCl | 1% ZnO | |
| **35% AFD (F)** | 100 mMolal CuAc | 100 mMolal ZnCl | 2% ZnO | |
| **35% AFD (B)** | 100 mMolal CuCl | 100 mMolal ZnCl | | Lauric acid 5% |
| **35% AFD (B)** | 50 mMolal CuAc | 100 mMolal ZnCl | | Decanoic acid 5% |
| **36% AFD (B)** | 89 mMolal CuAc | 89 mMolal ZnCl | 11% Kaolin | 10.7% PEG-400, 4.5% glycerol |
| **40% AFD (B)** | 100 mMolal CuAc | 100 mMolal ZnCl | 1% ZnO | 8% Triethyl citrate (TEC), |
| **40% AFD (B)** | 100 mMolal FeCl | | | 1.1% KOH |
| **40% AFD (B)** | 497 mMolal FeCl | | | |
| **40% AFD (B)** | 755 mMolal FeCl | | | |
| **40% AFD (B)** | 198 mMolal CuCl | | | |
| **40% AFD (B)** | 201 mMolal ZnCl | | | |
| **40% AFD (B)** | 599 mMolal ZnCl | | | |
| **40% AFD (B)** | 52 mMolal AICI | | | |
| **40% AFD (B)** | 50 mMolal AlCl | | | 0.5% KOH |
| **40% AFD (B)** | 64 mMolal MoAc | | | |
| **40% AFD (B)** | 100 mMolal CuAc | 100 mMolal ZnCl | | |
| **40% AFD (B)** | 100 mMolal CuAc | 100 mMolal ZnCl | | 4% lauric acid |
| **40% AFD (B)** | 100 mMolal CuAc | 100 mMolal ZnCl | | 1% beta-carotene |
| **40% AFD (B)** | 100 mMolal CuCl | 100 mMolal ZnCl | | 100 mMolal benzoic acid, 200 mMolal KOH |
| **40% AFD (B)** | 100 mMolal CuCl | | | |
| **40% AFD (B)** | 100 mMolal CuAc | | | |
| **40% AFD (B)** | 100 mMolal CuAc | 100 mMolal ZnCl | | |
| **40% AFD (B)** | 100 mMolal CuAc | 100 mMolal ZnCl | 1% ZnO | |
| **40% AFD (B)** | 100 mMolal CuAc | 100 mMolal ZnCl | | 12% PEG-400, 5% glycerol |
| **40% AFD (B)** | 100 mMolal ZnAc | 66 mMolal FeCl | | |
| **40% AFD (B)** | 100 mMolal ZnAc | 100 mMolal FeCl | | |
| **40% AFD (B)** | 50 mMolal CuCl | 100 mMolal ZnAc | 50 mMolal FeCl | |
| **40% AFD (B)** | 100 mMolal CuAc | 66 mMolal FeCl | | |
| **40% AFD (B)** | 100 mMolal CuAc | 100 mMolal FeCl | | |
| **40% AFD (B)** | 100 mMolal CuCl | 100 mMolal ZnCl | | 40 mMolal benzoic acid, |
| **.** | | | | 200 mMolal KOH |
| **40% AFD (B)** | 100 mMolal CuCl | 100 mMolal ZnCl | 1.6% ZnO | 40 mMolal benzoic acid, |
| **40% AFD (B)** | 100 mMolal CuCl | 100 mMolal ZnCl | | |
| **40% AFD (B)** | 100 mMolal CuCl | 100 mMolal ZnCl | | 50 mMolal KOH |
| **40% AFD (B)** | 100 mMolal CuCl | 100 mMolal ZnCl | | 100 mMolal KOH |
| **40% AFD (B)** | 100 mMolal CuCl | 100 mMolal ZnCl | | 200 mMolal KOH |
| **40% AFD (B)** | 150 mMolal CuAc | 100 mMolal ZnCl | | 4% lauric acid |
| **40% AFD (B)** | 100 mMolal CuAc | 100 mMolal ZnCl | | 0.1% patent blue |
| **40% AFD (B)** | 100 mMolal CuAc | 100 mMolal ZnCl | | 0.1% lissamine green |
| **40% AFD (B)** | 100 mMolal CuAc | 100 mMolal ZnCl | | 0.1% brilliant black |
| **40% AFD (B)** | 100 mMolal CuAc | 100 mMolal ZnCl | | 0.1% chlorophyllin |
| **40% AFD (B)** | 100 mMolal CuAc | 100 mMolal ZnCl | | 0.1% brilliant blue |
| **40% AFD (B)** | 250 mMolal CuCl | | | 4% Lauric acid, 1.1% KOH |
| **40% AFD (B)** | 125 mMolal CuCl | | | 1.1% KOH |
| **40% AFD (B)** | 100 mMolal ZnAc | | | |
| **40% AFD (B)** | 100 mMolal CuCl | 100 mMolal ZnAc | | |
| **40% AFD (B)** | 100 mMolal CuAc | 100 mMolal ZnCl | | 5% Hexylene glycol |
| **40% AFD (B)** | 100 mMolal CuCl | 100 mMolal ZnCl | | 100 mMolal Benzoic acid |
| **40% AFD (B)** | 100 mMolal CuCl | 100 mMolal ZnCl | | 500 mMolal Benzoic acid |
| **40% AFD (B)** | 100 mMolal CuAc | 100 mMolal ZnCl | | 5% Tributyrin |
| **40% AFD (B)** | 100 mMolal CuAc | 100 mMolal ZnCl | | 10% Tributyrin |
| **40% AFD (B)** | 100 mMolal CuCl | 100 mMolal ZnCl | 1% ZnO | 100 mMolal Benzoic acid, |
| **40% AFD (B)** | 100 mMolal CuCl | 100 mMolal ZnCl | | 100 mMolal Benzoic acid, 8% TEC |
| **40% AFD (B)** | 100 mMolal CuCl | 100 mMolal ZnCl | | 100 mMolal Benzoic acid, 5% Tributyrin |
| **40% AFD (B)** | 100 mMolal CuCl | 100 mMolal ZnCl | | 100 mMolal Benzoic acid, 50 mMolal KOH |
| **40% AFD (B)** | 100 mMolal CuCl | 100 mMolal ZnCl | | 100 mMolal Benzoic acid, 100 mMolal KOH |
| **40% AFD (B)** | 100 mMolal CuCl | 100 mMolal ZnCl | | 100 mMolal Benzoic acid, 200 mMolal KOH |
| **40% AFD (B)** | 100 mMolal CuCl | 100 mMolal ZnCl | | 200 mMolal |
| | | | | Benzoic acid, 200 mMolal KOH |
| **40% AFD (B)** | 100 mMolal CuCl | 100 mMolal ZnCl | 2% ZnO | 100 mMolal Benzoic acid, |
| **40% AFD (B)** | 100 mMolal CuCl | 100 mMolal ZnCl | 2% ZnO | 200 mMolal Benzoic acid, |
| **40% AFD (F)** | 100 mMolal CuAc | 100 mMolal ZnCl | 1% ZnO | |
| **40% AFD (F)** | 100 mMolal CuAc | 100 mMolal ZnCl | 2% ZnO | |
| **40% AFD (F)** | 100 mMolal CuAc | 100 mMolal ZnCl | | 6% Triethyl citrate (TEC) |
| **40% AFD (F)** | 100 mMolal CuAc | 100 mMolal ZnCl | | 12% Triethyl citrate (TEC) |
| **40% AFD (F)** | 100 mMolal CuAc | 100 mMolal ZnCl | | 6% PEG 400 |
| **40% AFD (F)** | 100 mMolal CuAc | 100 mMolal ZnCl | | 12% PEG 400 |
| **40% AFD (F)** | 100 mMolal CuAc | 100 mMolal ZnCl | | 12% TEC, 5% decanoic acid |
| **40% AFD (F)** | 100 mMolal CuAc | 100 mMolal ZnCl | | 12% TEC, 5% glycerol |
| **40% AFD (F)** | 100 mMolal CuAc | 100 mMolal ZnCl | 5% nano ZnO | 12% TEC |
| **40% AFD (F)** | 100 mMolal CuAc | 100 mMolal ZnCl | | 12% PEG, 5% decanoic acid |
| **40% AFD (F)** | 100 mMolal CuAc | 100 mMolal ZnCl | | 12% PEG, 5% glycerol |
| **40% AFD (F)** | 100 mMolal CuAc | 100 mMolal ZnCl | 5% nano ZnO | 12% PEG |
| **40% AFD (F)** | 100 mMolal CuAc | 100 mMolal ZnCl | | 8% Triethyl citrate (TEC) |
| **40% AFD (F)** | 100 mMolal CuAc | 100 mMolal ZnCl | 1% ZnO | 8% Triethyl citrate (TEC) |
| **40% AFD (F)** | 100 mMolal CuAc | 100 mMolal ZnCl | 2% ZnO | 8% Triethyl citrate (TEC) |
| **40% AFD (F)** | 100 mMolal CuAc | 100 mMolal ZnCl | | 8% Triethyl citrate (TEC), 5% Decanoic acid |
| **40% AFD (F)** | 100 mMolal CuAc | 100 mMolal ZnCl | | |
| **40% AFD (F)** | 50 mMolal CuAc | 50 mMolal CuCl | 100 mMolal ZnCl | |
| **40% AFE 40% AFE** | 50 mMolal ZnCl 20% Kaolin | | | |
| **40% AFW (F)** | 100 mMolal CuAc | 100 mMolal ZnCl | | |
| **40% S (F)** | 100 mMolal CuAc | 100 mMolal ZnCl | | |
| **41% AFD (B)** | 390 mMolal ZnCl | | | |
| **42% AFD (B)** | 1.6% Kaolin | 100 mMolal CuCl | | |
| **43% AFD (B)** | 1.6% Kaolin | | | |
| **43% AFD (B)** | 383 mMolal CuCl | | | |
| **45% AFD (B)** | 229 mMolal FeCl | | | |
| **50% AFD (B)** | 207 mMolal FeCl | | | |
| **50% AFD (B)** | 68 mMolal MoAc | | | |
| **50% AFD (F)** | 80 mMolal CuAc | 20 mMolal CuCl | 100 mMolal ZnCl | |
| **50% AFD (F)** | 50 mMolal CuAc | 50 mMolal CuCl | 100 mMolal ZnCl | |
| **50% AFD (F)** | 100 mMolal CuAc | 100 mMolal ZnCl | | 8% Triethyl citrate (TEC) |
| **50% AFD (F)** | 100 mMolal CuAc | 100 mMolal ZnCl | | 8% Triethyl citrate (TEC), 5% Decanoic acid |
| **50% AFD (F)** | 100 mMolal CuAc | 100 mMolal ZnCl | 2% ZnO | 10% Triethyl citrate (TEC), 5% Decanoic acid |
| **50% AFD (F)** | 100 mMolal CuCl | 100 mMolal ZnCl | 2% ZnO | 10% Triethyl citrate (TEC), 5% Decanoic acid |
| **50% AFD (F)** | 100 mMolal CuAc | 100 mMolal ZnCl | | 10% Triethyl citrate (TEC), 5% Decanoic acid |
| **50% AFD (F)** | 100 mMolal CuAc | 100 mMolal ZnCl | | |
| **50% AFW (F)** | 100 mMolal CuAc | 100 mMolal ZnCl | | |
| **50% S (F)** | 100 mMolal CuAc | 100 mMolal ZnCl | | |
| **50% S (F)** | 50 mMolal CuAc | 50 mMolal CuCl | 100 mMolal ZnCl | |
| **50% S (F)** | 80 mMolal CuAc | 20 mMolal CuCl | 100 mMolal ZnCl | |
| **60% AFD (B)** | 100 mMolal CuCl | 100 mMolal ZnCl | | |
| **60% AFD (F)** | 50 mMolal CuCl | 50 mMolal CuAc 100 | 100 mMolal ZnCl | |
| **60% AFD (F)** | 100 mMolal CuCl | 100 mMolal ZnCl | | |
| ^{a}No ethanol was added in the formulation using an acetone:water mixture as the solvent. | | | | |

### Example 3. Impact of concentration on compatibility

### Example 3.1

A solution of 70% shellac in ethanol was prepared which resulted in a clear amber viscous solution. Upon 5-fold dilution in ethanol a free flowing clear yellow solution was obtained with no visible precipitates.

### Example 3.2

200 mMolal copper acetate was dissolved in ethanol producing a dark blue/green solution. It was diluted 1:10 in ethanol producing a clear pale blue solution. If, however, it is diluted 1:10 in a 17% shellac solution, a turbid greenish solution with copious precipitate is produced.

### Example 3.3

Solid shellac can be added to an ethanolic copper acetate solution (100 mMolal) to achieve a homogenous solution comprising 40% shellac with no visible precipitate.

Concentrated ethanolic solutions of shellac can be diluted in ethanol easily. However, ethanolic solutions of shellac plus copper acetate form shellac precipitates when diluted. This is surprising because the high concentration material is stable whereas the low concentration is not. Additionally, this is advantageous because: (1) these solutions can be produced at high concentrations such that thick barriers can be formed and (2) because upon dilution (which will happen on application) the material can precipitate in *situ* thus forming a barrier faster.

### Example 4. Retention testing of Rosin vs Shellac with and without metals

### Materials

Copper acetate and zinc chloride were weighed into falcon tubes and dissolved in ethanol. Next shellac, or rosin were also added and let dissolve overnight on a roller mixer. Equivalent materials without copper acetate and zinc were prepared by simple dissolving shellac (40 and 60%) or rosin (40%) in ethanol overnight using a roller mixer. Table 2 lists the materials prepared and their compositions.

**Table 2. Materials prepared for testing**

| **Resin** | **Conc. (wt%)** | **Cu Acetate (mMolal)** | **Zinc Chloride (mMolal)** |
|---|---|---|---|
| **Shellac** | 40 | | |
| **Shellac** | 60 | | |
| **Shellac** | 40 | 100 | 100 |
| **Rosin** | 40 | | |
| **Rosin** | 40 | 100 | 100 |

### Retention Testing

### Set up

Moistened carpet squares (5.5cm x 5.5cm) with a secondary backing of bitumen (i.e. underside; details in table below) were used as an approximation to the environment encountered in moist cow's hooves. Briefly, squares were dipped in a beaker with tap water for at 5-10 seconds and then fixed on a stand using a clamp on a vertical angle of 27° with the underside (i.e. bitumen backing) upwards.

**Table 3. Details of surface used for retention testing**

| | |
|---|---|
| Manufacturer | Vitrex |
| Product code | CT503 |
| Yarn | 100% polypropylene |
| Secondary backing | Polyester |
| Final backing | Polyester |
| Pile height | 3.5 mm ± 0.5 mm |

### Application

A new carpet surface was used per test. Compositions were applied with a plastic syringe (5 mL) always on the same area. The amount applied and the fraction subsequently lost (i.e. fraction which did not adhere to the carpet) was determined gravimetrically (Table 4).

**Table 4. Retention results**

| **Material** | **Material Applied, g** | **Material Lost, q** | **Material Loss %** |
|---|---|---|---|
| **40% Shellac** | 3.0 | 1.0 | 33% |
| **60% Shellac** | 3.0 | 1.5 | 50% |
| **40% Shellac + CuAc + ZnCl₂** | 3.0 | 0.5 | 17% |
| **40% Rosin** | 3.0 | 1.3 | 43% |
| **40% Rosin +CuAc + ZnCl₂** | 3.0 | 1.1 | 37% |

### Observations

The composition with 40% shellac with CuAc and ZnCl formed the best barrier. The second best was 60 % shellac. The other materials were not very viscous and formed a thin and fragile barrier.

### Conclusions

Surprisingly, the addition of antimicrobial metal enabled the use of lower amounts of shellac whilst achieving increased viscosity and improved retention. The results of these experiments showed only the compositions including the metal active and shellac had the necessary viscosity and adhesive properties for forming self-supporting hydrophobic barriers with adhesive properties. In contrast, the addition of metal actives to rosin failed to provide a practical barrier.

### Example 5. Viscosity enhancement

The present inventors found out that metals can also be used to induce viscosity increases in shellac compositions. The table below (extracted from main compilation table) provides a few examples of compositions with enhanced viscosity through the addition of metals. For the avoidance of doubt, this table is for illustration purposes only, as enhanced viscosity can be achieved with different metal combinations and/or concentrations.

**Table 5. Examples of enhanced viscosity materials (from Table 1).**

| **Shellac** | **Metal 1** | **Metal 2** | **Metal 3** | **Additives** |
|---|---|---|---|---|
| **40% AFD (B)** | 100 mMolal ZnAc | 66 mMolal FeCl | | |
| **40% AFD (B)** | 100 mMolal ZnAc | 100 mMolal FeCl | | |
| **40% AFD (B)** | 50 mMolal CuCl | 100 mMolal ZnAc | 50 mMolal FeCl | |
| **40% AFD (B)** | 125 mMolal CuCl | | | 1.1% KOH |
| **40% AFD (B)** | 100 mMolal FeCl | | | 1.1% KOH |
| **40% AFD (B)** | 50 mMolal AlC l | | | 0.5% KOH |
| **50% AFD (B)** | 68 mMolal MoAc | | | |

### Example 6. Resistance to alkali degradation

Shellac compositions are soluble under alkaline conditions (pH> 7). This limits their usefulness for topical barriers since physiological fluids are generally above pH 7.0. Additionally, the external environment, particularly in veterinary applications is often alkaline. In the case of digital dermatitis, barriers have to withstand contact with manure slurries which are usually alkaline (up to pH 9.0; see Salazar et al. Characterization of dairy slurry in southern Chile farms. Agricultura Tecnica, 67(2), 155, 2007; Fordham & Schwertmann, Composition and Reactions of Liquid Manure (Gülle), with Particular Reference to Phosphate: III. pH-Buffering Capacity and Organic Components 1. Journal of Environmental Quality, 6(2), 140-144, 1977; and UC Manure Technical Guide Series; Dairy Manure Nutrient Content and Forms). Contact with such solutions would result in rapid loss of shellac barrier integrity.

The present inventors have discovered that addition of metals dramatically increases the resistance of barriers to simulated slurry (SS). Surprisingly, the compositions described herein vastly outperformed metal-free barriers containing higher content of shellac (Table 6). This feature in the present invention is advantageous economically in that much lower content of shellac can be used thus resulting in lower manufacturing costs.

Furthermore, the present inventors found that wet barriers (i.e. before solvent evaporation) from the present compositions had very high resistance to SS. In contrast shellac only barriers that were not allowed to dry for 24 h performed very poorly. Resistance to alkali by wet barriers is particularly advantageous in a farm environment since the hoof will contact wet slurry soon after application of the barrier.

**Table 6. Barrier survival of compositions exposed to simulated slurry (SS). SS was either applied to wet barriers (as produced) or dry barriers (allowed to dry for 24 h at room temperature) and mass recovery was determined.**

| | Ethanolic shellac-only compositions | | | Ethanolic shellac composition plus metal |
|---|---|---|---|---|
| | 40% Shellac (w/w) | 50% Shellac (w/w) | 60% Shellac (w/w) | S1B2 (comprises 40% shellac) |
| Wet | 1.5 | 3.8 | 27.9 | 92.7 |
| Dry | 27.3 | 23.3 | 40.8 | 89.4 |

### Methodology

SS consisted of 75 mmolal ammonium carbonate (pH 8.9) as this was previously shown to be within the chemical range of faecal slurries at cattle farms (see Fordham and Schwertmann et al., and UC manure Technical Guide series, supra).

1.0 g of shellac composition was added to the bottom of a tube with 22 mL total capacity. 20 g of freshly prepared SS were either added immediately after ("wet") or after a 24 hour drying period (open tube at room temperature; "dry"). Exposure of barrier to SS was carried out for 24 h in a roller mixer at room temperature, after which the SS fraction was disposed of and mass of the remaining barrier was determined. Recovery was calculated in relation to controls not exposed to SS.

### Example 7. Antibiotic lock-In

The present compositions are effective at preventing loss of antibiotic to surrounding environment. This is illustrated in Example 7.1 where a solution of oxytetracycline (0.5%) was applied to a simulated lesion surface. In absence of barrier, and despite being exposed to only to 1 ml of fluid, 42% of antibiotic were lost from the simulated lesion surface within 90 minutes. In contrast, when a protective barrier was applied over the antibiotic, less than 0.2% oxytetracycline was lost to the surrounding fluid. In Example 7.2, a chlortetracycline spray (commonly used to treat digital dermatitis) was applied to bovine cadaver limbs (between the interdigital cleft and the dew claws) and exposed to simulated slurry for an hour. In the absence of a barrier (n=4), substantial losses were observed (93% ±3.6%). Applying a barrier (n=4), reduced losses (5 fold) to 18% ±3.5%.

### Example 7.1

Antibiotic loss experiments were carried out in 24 well plates. 1 ml of a 10 % bovine gelatine solution (prepared at 50°C) was added to the bottom of each well and allowed to settle for 90 min at 7± 2°C. Next 20 µL of an ethanolic solution of 0.5% oxytetracycline was added to the bottom of each well and allowed to dry for 5-10 minutes. Half of the experimental wells (N=6 for each arm) had then 0.50±0.05 g of S1B2 added (prepared as per example 1.2) thus forming a protective barrier over the antibiotic and were allowed to dry for 5-10 min (appropriate OTC free wells were also prepared for background correction). Next 1 mL of UHP water was added to all wells. These were kept in the dark for 90 min after which samples of the aqueous fraction were collected for fluorescence measurement in a plate reader (355/590) against oxytetracycline standards (curve fitting by Graphpad). Antibiotic losses to the aqueous phase were determined as 42% for antibiotic only and 0.1% for antibiotic plus barrier.

### Example 7.2

*Ex vivo* antibiotic loss experiments were carried out with bovine cadaver limbs. An antibiotic spray suspension used for treating Digital Dermatitis (Animedazon Spray, containing chlortetracycline as the active and Patent Blue V as a dye) was applied to each limb by spraying an area commonly afflicted with lesions (the region between the interdigital cleft and dew claws) for 2 seconds. The spray-can mass was recorded before and after each application to control for the quantities of spray applied. Applied antibiotic spray was allowed to dry for 60 seconds and then limbs were either: (a) Control Group (n=4): directly immersed in 1 litre of simulated slurry (SS; 75 mM ammonium carbonate) or (b) Barrier Group (n=4): treated with a barrier formulation (15 ±5 g of S1B2 from example 1.2) applied to cover the antibiotic spray-site, allowed to dry for a further 120 seconds and then immersed in 1 litre of SS. After 1 hour of immersion, 10 mL samples of SS were taken for each limb and the assay terminated. Samples were analysed for absorbance to determine the proportion of antibiotic formulation lost to the simulated slurry fluid; the strong and characteristic spectrochemical profile of Patent Blue V (λ max_{H2O} = 639 nm [J. Chem. Sci. (2018) 130:12]) made absorbance measurements a convenient proxy for antibiotic losses. Absorbance standards were prepared by serial dilution of weighed masses of antibiotic spray in the simulated slurry solution. Subsequently, sextuplicate aliquots (200 µL each) from each standard and assay sample were plated (Corning Co-star 96 well) and measured for absorbance between 350 and 850 nm. Raw spectra had somewhat noisy and variable baselines (due to organic contaminants on the limbs) and required processing in MATLAB (Savitsky Golay filtering followed by a baseline subtraction with second order polynomials) to obtain reliable absorbance maxima. Finally, linear standard curves (concentration vs absorbance at 639 nm) were used to generate loss data for assay samples. In the absence of barrier protection, 93% of applied Patent Blue was lost to the assay fluid; when a barrier formulation was used, losses were reduced more than 5 fold to 18%.

### Example 8. Contact killing and metal release by barriers

Many of the compositions disclosed herein release very low levels of metal when in contact with fluid. Despite this, these compositions are bactericidally active on contact. In contrast, shellac-only barriers were found to have no measurable contact killing activity.

### Contact Killing/Metal Release Methodology

1 mL of each material was transferred into a well on 12 well-plates and allowed to settle overnight. On the following day, 1ml of E. *coli* culture in LB (~10⁶-10⁷ CFU/ml) was added on top of each material and incubated for 24h at 30°C under mild agitation (100rpm). After 24 hours, samples from the bacterial medium were collected to quantify bacterial concentration (agar plate counting) and metal release (ICP-OES).

### Materials tested in contact killing and metal release

**Table 7. Materials tested in the assays.**

| ***Label*** | ***Shellac (%)*** | ***Copper*** | ***Zinc*** | ***Additives*** |
|---|---|---|---|---|
| F0 | 40 | - | - | - |
| F1 | 27 | 150mM CuAc | | 3% TEC |
| F3 | 40 | 100mM CuAc | - | - |
| F4 | 40 | 100mM CuAc | 100mM ZnCl₂ | |
| F6 | 40 | 100mM CuAc | 100mM ZnCl₂ | 1% ZnO; 8% TEC |
| S7 | 40 | 100mM CuAc | 100mM ZnCl₂ | 12% PEG; 5% Glycerol |
| F8 | 30 | 150mM CuAc | - | - |
| F11 | 40 | - | 100mM ZnAc | 66mM FeCl₃ |
| F13 | 40 | 50mM CuCl₂ | 100mM ZnAc | 50mM FeCl₃ |
| F14 | 40 | 100mM CuAc | - | 66mM FeCl₃ |
| F16 | 40 | 100mM CuCl₂ | 100mM ZnCl₂ | 40mM Benz. ac; 200mM KOH |
| F26 | 40 | 100mM CuAc | 100mM ZnCl₂ | 0.1% Patent Blue |
| F27 | 40 | 100mM CuAc | 100mM ZnCl₂ | 0.1% Lissamine green |
| F30 | 40 | 100mM CuAc | 100mM ZnCl₂ | 0.1% Brilliant blue |

### Metal Release Results

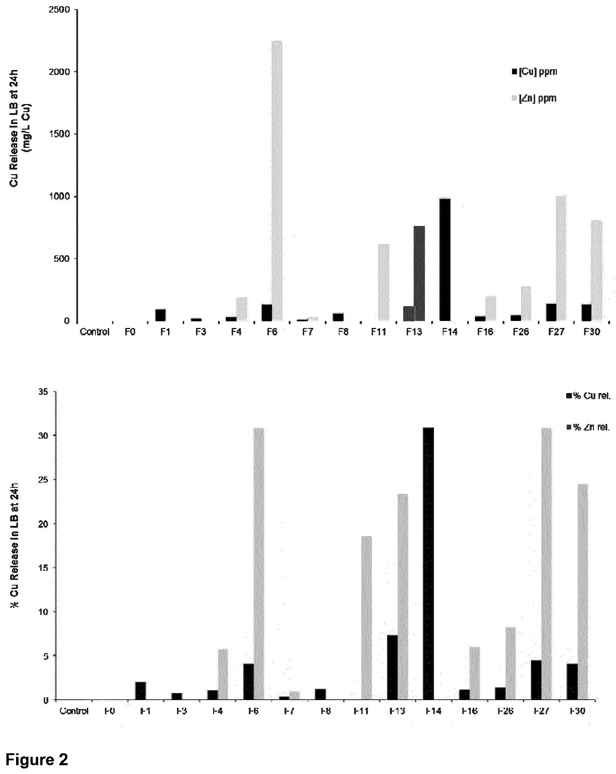

Samples collected directly from contacting killing assay after 24h in LB (bacterial medium) at 30°C under mild agitation. Figure 2 shows the release for Zn and Cu from barriers. Top graph refers to absolute concentrations (ppm) whereas bottom graph refers to relative release.

### Contact Killing Results

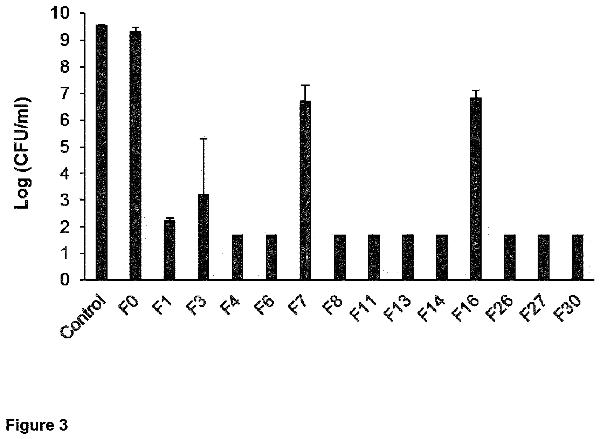

Figure 3 shows the contact killing results Log CFU/ml after 24h incubation with *E*. *coli* K12 in LB. F0, which consists of a shellac-only composition, had the same bacterial growth as control experiment (no shellac). 1.7 Log CFU/ml was the limit of detection and therefore for the materials that had this result (F4, F6, F8, F11, F13, F14, F26, F27 and F30) no bacterial colonies were formed.

### Example 9. Application in cows with digital dermatitis

S1B2 was applied to eight hooves of dairy cows suffering from digital dermatitis. Briefly, hooves were immobilised and water-hosed to remove slurry and other detritus. Chlortetracycline was applied (sprayable formulation) and allowed to dry for a few seconds. Subsequently, 5 to 10 g of S1B2 was applied such that the digital dermatitis lesion was fully covered. After one minute, the cow was released and allowed back into the yard where the hooves were in contact with slurry and immersed in a formaldehyde bath several times a day (after the cow was robotically milked). Two days post-application, barrier integrity was assessed for six hooves, of these four had near full wound coverage, one had some barrier residues and one had no visible barrier. In contrast, by day seven, only one of seven hooves assessed had any residue - the other six had no visible barrier at all.

### Example 10. Application strategies

Several formulations (Table 8) were applied on the hooves of dairy cows suffering from digital dermatitis. The products were applied, as per example 9, using various application strategies.

**Table 8.**

| **Material** | **Applicator** | **Pre-applied actives** |
|---|---|---|
| **S1B2** | Squeezy Bottle | Chlortetracycline |
| **S1B2** | Syringe | Chlortetracycline |
| **S1B2** | Brush | Washed with chlorhexidine followed by chlortetracycline |
| **S1B3** | Squeezy Bottle | Chlortetracycline |
| **S1B4** | Squeezy Bottle | Chlortetracycline |
| **S1B5** | Squeezy Bottle | Chlortetracycline |
| **S1B6** | Single Barrel Gun | Chlortetracycline |
| **S1B8** | Syringe | Chlortetracycline |
| **S1B9** | Syringe | Chlortetracycline |

### Example 11. Application on ringworm lesions

S1B2 was applied on two ring worm lesions in a dairy cow. 5-10 g of S1B2 were directly applied on each wound (i.e. no antibiotic applied). After 7 days the barrier was completely absent from one lesion (in the thigh area), but partial coverage (c.a. 50% of the lesion area) was observed in the other lesion (in the back section of the cow).

### Example 12. Use in udder infections

A cow presenting extensive ulceration in its udder was treated with S1B2. The lesion was first washed and disinfected with 0.5% chlorhexidine. Chlortetracycline spray was applied and then the lesion was covered with S1B2 using a brush. No obvious losses of product were observed during application.

### Example 13. Use in udder infections

The experiments set out in Example 12 were repeated using the S1B1 composition instead of S1B2.

### Example 14. Use in udder infections

The experiments set out in Example 12 were repeated with the proviso that the lesion was only wiped with a paper towel and chlortetracycline spray was then applied (i.e. chlorhexidine was not used). After 3 days, circa 75% of the lesion area was still covered by the barrier and the lesion showed visible improvements.

### Example 15. Clinical improvement in the mobility of treated cows

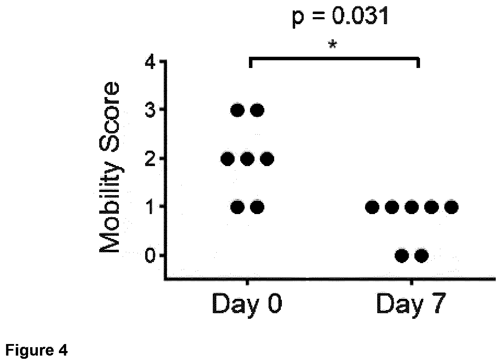

Cows afflicted with digital dermatitis were treated with chlortetracycline spray and then S1B2 barrier was added as per example 9. Their mobility (lameness) was assessed by veterinarians on the day of treatment (day 0) and after 7 days, using a standard mobility scoring system (Griffiths et al, 2018), described in Table 9. Results are shown in Figure 4. In this system, lower scores denote better mobility; at day 0, mobility scores varied from 1 (imperfect) to 3 (severely impaired). By day 7, there was a statistically significant improvement in the mobility scores, which had normalised to 0 (good mobility) or 1.

**Table 9. Description of the mobility scoring system (Griffiths et al, 2018).**

| **Score** | **Mobility Category** | **Description** |
|---|---|---|
| **0** | Good mobility | Walks with even weight bearing and rhythm on all four feet, with a flat back. |
| | | Long, fluid strides possible |
| **1** | Imperfect mobility | Steps uneven (rhythm or weight bearing) or strides shortened; affected limb or limbs not immediately identifiable. |
| **2** | Impaired mobility | Uneven weight bearing on a limb that is immediately identifiable and or/obviously shortened strides (usually with an arch to the centre of the back). |
| **3** | Severely impaired mobility | Unable to walk as fast as a brisk human pace (cannot keep up with the healthy herd) and signs of score 2 |

### Methods statement for mobility work

Animal mobility was assessed by veterinarians using a standard scoring system described in Table 9. Single-tailed Wilcoxon matched-pairs signed-rank test was employed to test for statistical significance. A value of P < 0.05 was considered statistically significant. Mobility scoring is described in Griffiths, B. E., Grove White, D. & Oikonomou, G. A Cross-Sectional Study Into the Prevalence of Dairy Cattle Lameness and Associated Herd-Level Risk Factors in England and Wales. Front. Vet. Sci. 5, 65 (2018).

## Claims

1. A liquid dressing composition for veterinary use in the treatment or prevention of infection and/or wounds, wherein the composition comprises shellac, an anti-infective metal-based active selected from one or more of Cu, Zn, Fe, Al, Mo and Ca, wherein where the anti-infective metal-based active is based on zinc it is selected from one or more of zinc chloride and zinc acetate or zinc carboxylate complexes, and a volatile solvent, wherein the composition is capable of forming a barrier when topically applied to a subject, wherein the composition is for veterinary administration and topically applied to a subject does not include application to the teeth of the subject.

2. The liquid dressing composition for use in the treatment or prevention of infection and/or wounds of claim 1, wherein the liquid dressing composition is applied to a site of a wound or infection of the subject or which is at risk of developing a wound or infection.

3. The liquid dressing composition for use in the treatment or prevention of infection and/or wounds of claim 1 or claim 2, wherein the anti-infective metal-based active comprises an anti-infective metal-based active selected from one or more of Cu, Zn, Fe, Al and Mo.

4. The liquid dressing composition for use in the treatment or prevention of infection and/or wounds of claim 1 or claim 2, wherein the barrier is a hydrophobic barrier.

5. The liquid dressing composition for use in the treatment or prevention of infection and/or wounds of any one of the preceding claims, wherein the composition is capable of forming a self-supporting hydrophobic barrier when topically applied to a subject.

6. The liquid dressing composition for use in the treatment or prevention of infection and/or wounds of any one of the preceding claims, wherein the composition is for use in the treatment or prevention of bacterial, fungal, parasitic or viral infection, optionally wherein the infection is caused by a gram-negative or a gram-positive bacterium.

7. The liquid dressing composition for use in the treatment or prevention of infection and/or wounds of claim 6, wherein:
(a) the bacterial infection is caused by a Spirochaetes sp., an *Escherichia sp.,* a *Staphylococcus sp.,* a *Bacillus sp.,* a *Pseudomonas sp.,* a *Vibrio sp., a Streptococcus sp.,* a *Klebsiella sp.,* a *Micrococcus sp.,* a *Clostridium sp.,* an *Acinetobacter sp.,* a *Mycobacterium sp.,* a *Salmonella sp.,* a *Chlamydia sp.,* a *Erysipelothrix sp.,* a *Corynebacterium sp., a Leptospira sp., a Cutibacterium sp, a Gardnerella* sp., a *Campylobacter sp.,* a *Klebsiella sp., a Capnocytophaga sp., a Vibrio sp., a Pasteurella sp.,* a *Aeromonas sp., a Eikenella sp., a Mycoplasma sp., a Calymmatobacterium sp., a Helicobacter sp., a Clostridium sp., a Listeria sp., a Treponema sp., a Bartonella sp., a Nocardia sp., a Yersinia sp.,* a *Serratia sp., a Burkholderia sp., a Actinomyces sp., a Borrelia sp.,* or an *Enterococcus sp; or* (b) the fungal infection is caused by a *Candida sp.,* a *Malassezia sp., a Trycophyton sp., a Microsporum* sp., a *Epidermophyton Sp., a Fusarium sp., a Rhizopus* sp., a *Rhizomucor sp., a Mucor* sp., a *Syncephalastrum sp., a Cunninghamella sp., a Apophysomyces sp., a Lichtheimia* sp., a *Saksenaea sp.,* an *Aspergillus sp., a Blastomyces sp., a Coccidioides sp., a Cryptococcus sp., a Histoplasma sp., a Cochliobolus sp., a Cladophialophora sp., a Exophiala sp., a Madurella sp., a Pyrenochaeta sp., a Pneumocystis sp., a Paracoccidioides sp., a Sporothrix sp.,* or a *Talaromyces sp;* or
(c) the parasitic infection is caused by a *Sarcoptes sp., Leishmania sp.,* Ancylostomatidae such as *Ancylostoma sp.* or *Necator sp., a Strongyloides sp., a Onchocerca sp.,* dipterous larvae, *Protophormia terraenovae, Cochliomyia hominivorax, Cordylobia anthropophaga,* or *Sarcophaga bercaea, a Balamuthia sp* or a *Acanthamoeba sp;* or
(d) the viral infection is caused by a Papillomavirus, a enterovirus such as Coxsackievirus, Molluscipoxvirus sp., Varicellovirus, or a herpesvirus; or.
(e) the infection is caused by an Adenoviridae virus, a Caliciviridae virus, a Coronaviridae virus, a Flaviviridae virus, a Herpesviridae virus, an Orthomyxoviridae virus, a Papillomaviridae virus, a Paramyxoviridae virus, a Parvoviridae virus, a Poxviridae virus, a Reoviridae virus or Phenuiviridae virus.

8. The liquid dressing composition for use in the treatment or prevention of infection and/or wounds of any one of the preceding claims, wherein the treatment comprises applying a second active agent to a site on the subject and applying the liquid dressing composition over the site to lock in the second active agent, wherein the second active agent comprises an antibiotic agent, an antimicrobial agent and/or a haemostatic active or wherein the second active agent comprises iodine.

9. The liquid dressing composition for use in the treatment or prevention of infection and/or wounds of any one of claims 6 to 8, wherein the self-supporting hydrophobic barrier reduces the loss of the second active agent by at least 20 %, and optionally by at least 30%, as compared to treatment without the application of the self-supporting hydrophobic barrier.

10. The liquid dressing composition for use in the treatment or prevention of infection and/or wounds of any one of the preceding claims, wherein the liquid dressing composition is for the treatment of cows, buffalos, yak, pigs, horses, sheep, goats, llamas, alpacas, deer, donkeys, zebus, zebras, elephants, orangutans, chimpanzees, gorillas, lemurs, gibbons, baboons, chickens, turkeys, ducks, emus, geese, ostrich, cats, dogs, ferrets, gerbils, hamsters, chinchillas, rats, rabbits, parrots, canaries, galliformes, anseriformes or passeriformes.

11. The liquid dressing composition for use in the treatment or prevention of infection and/or wounds of any one of the preceding claims, wherein the liquid dressing composition is applied as a paint composition, optionally having a viscosity at 25°C of between 1000 cP and 5000 cP, optionally wherein the liquid dressing composition is applied as a spray composition, optionally having a viscosity at 25°C of between 10 cP and 3000 cP.

12. The liquid dressing composition for use in the treatment or prevention of infection and/or wounds of any one of the preceding claims, wherein the liquid dressing composition is for the treatment of bovine digital dermatitis, udder infection, leishmaniosis, diabetic ulcers or Buruli ulcers, teat sealing, horn removal, udder dermatitis, post amputation, foot rot in sheep or mud fever in horses.

13. The liquid dressing composition for use in the treatment or prevention of infection and/or wounds of any one of the preceding claims, wherein:
(a) the shellac is wax-free shellac, de-waxed shellac, dewaxed bleached Shellac, dewaxed and decolourised shellac, shellac ester or waxed shellac; and/or
(b) the composition comprises 10 to 70 % w/w shellac and optionally 40% w/w to 60% w/w shellac; and/or
(c) the solvent is ethanol, a acetone-water mixture, isopropanol, propanol and butanol, or combinations thereof; and/or
(d) the anti-infective metal-based active is copper chloride, copper acetate, copper oxide, copper oxo-hydroxide, copper hydroxide, zinc chloride, zinc acetate, iron chloride , aluminium chloride, molybdenum acetate or kaolin.

14. The liquid dressing composition for use in the treatment or prevention of infection and/or wounds of any one of the preceding claims, wherein:
(a) the anti-infective metal active is present at a concentration of 20 mMolal to 500 mMolal; and/or
(b) the addition of the anti-infective metal-based active increases the viscosity of the composition and/or the thickness of the barrier over a composition comprising shellac alone; and/or
(c) the antimicrobial composition is co-administered with one or more antibiotics, optionally wherein the composition is applied to a subject at a location where the antibiotic has been applied; and/or
(d) the liquid dressing composition comprises or is co-administered with an antibiotic, an antifungal agent, and/or a disinfectant; and/or
(e) the barrier formed when the composition is applied to a subject is resistant to environments having a pH greater than pH 9.0; and/or
(f) the liquid dressing composition comprises a further resin component; and/or
(g) the liquid dressing composition further comprises fibres, preferably between 1 and 15% w/w, more preferably between 2.5 and 10%, most preferably between 2.5% and 7.5 %, optionally wherein fibres are cellulose fibres.

15. The liquid dressing composition for use in the treatment or prevention of infection and/or wounds of any one of the preceding claims, wherein the liquid dressing composition further comprises one or more hydrophilic additives as plasticisers and/or to accelerate formation of the self-supporting hydrophobic barrier, optionally wherein the hydrophilic additives are selected from TEC, a polyalkylene glycol such as a polyethylene glycol, glycerol, hexylene glycol, and/or triacetin.

16. The liquid dressing composition for use in the treatment or prevention of infection and/or wounds of any one of the preceding claims, wherein the liquid dressing composition further comprises one or more hydrophobic barrier enhancing agents to enhance water resistance, optionally wherein the antimicrobial composition hydrophobic barrier enhancing agent is selected from decanoic acid, octanoic acid, oleic acid and/or lauric acid.

17. The liquid dressing composition for use in the treatment or prevention of infection and/or wounds of any one of the preceding claims, wherein the composition further comprises one or more buffering agents to inhibit or reduce barrier degradation, optionally wherein the buffering agent is a di-carboxylic acid, optionally wherein the dicarboxylate is adipic acid, azelaic acid, succinic acid, pimelic acid or decanoic acid.

18. The liquid dressing composition for use in the treatment or prevention of infection and/or wounds of any one of the preceding claims, wherein the antimicrobial composition further comprises an anti-inflammatory agent or a complexing agent to enhance loading or solubility of the anti-infective metal-based active, optionally wherein the anti-inflammatory agent or the complexing agent is selected from salicylic acid, sodium salicylate and/or propionic acid.

19. The liquid dressing composition for use in the treatment or prevention of infection and/or wounds of any one of the preceding claims, wherein the liquid dressing composition further comprises a haemostatic agent wherein the haemostatic agent enhances clotting action for use in acute wound care.

20. The liquid dressing composition for use in the treatment or prevention of infection and/or wounds of any one of the preceding claims, wherein the composition is non-sprayable having droplet sizes of 1 mm or greater.

21. A device for applying a liquid dressing composition for veterinary use in the treatment of infection and/or wounds, the device comprising:
a first chamber containing a composition comprising shellac and an anti-infective metal-based active selected from one or more of Cu, Zn, Fe, Al, Mo and Ca in a volatile solvent for use as defined in any one of claims 1 to 20 and a second chamber containing a non-solvent;
a mixing chamber in communication with the first and second chambers;
actuating means for delivering the composition and non-solvent to the mixing chamber to provide a mixed composition, wherein the non-solvent is glycerol or a polyalkylene glycol; and
an applicator in communication with the mixing chamber for applying the mixed composition to a location on a subject, wherein the composition is capable of forming a hydrophobic barrier when topically applied to a subject, wherein the composition is for veterinary administration and where topically applied to a subject does not include application to the teeth of the subject.

22. The device of claim 21, wherein the polyalkylene glycol is PEG, and/or wherein the device is a dual cartridge applicator.

23. A pressurised spray delivery device comprising a composition comprising shellac and a metal active in a volatile solvent for use as defined in any one of claims 1 to 20 and a propellant for delivering the composition.

## Patentansprüche

1. Flüssige Verbandszusammensetzung zur veterinärmedizinischen Verwendung bei der Behandlung oder Prävention einer Infektion und/oder von Wunden, wobei die Zusammensetzung Schellack, einen infektionshemmenden Wirkstoff auf Metallbasis, ausgewählt aus einem oder mehreren aus Cu, Zn, Fe, Al, Mo und Ca, wobei, wenn der infektionshemmende Wirkstoff auf Metallbasis auf Zink basiert, dieser aus einem oder mehreren aus Zinkchlorid und Zinkacetat oder Zinkcarboxylat-Komplexen ausgewählt ist, und ein flüchtiges Lösungsmittel umfasst, wobei die Zusammensetzung dazu in der Lage ist, eine Barriere zu bilden, wenn sie topisch auf ein Individuum angewendet wird, wobei die Zusammensetzung zur veterinärmedizinischen Verabreichung dient und die topische Anwendung nicht die Anwendung auf die Zähne des Individuums umfasst.

2. Flüssige Verbandszusammensetzung zur Verwendung bei der Behandlung oder Prävention einer Infektion und/oder von Wunden nach Anspruch 1, wobei die flüssige Verbandszusammensetzung auf eine Stelle einer Wunde oder Infektion des Individuums, oder bei dem ein Risiko zur Entwicklung einer Wunde oder Infektion besteht, aufgebracht wird.

3. Flüssige Verbandszusammensetzung zur Verwendung bei der Behandlung oder Prävention einer Infektion und/oder von Wunden nach Anspruch 1 oder Anspruch 2, wobei der infektionshemmende Wirkstoff auf Metallbasis einen infektionshemmenden Wirkstoff auf Metallbasis, ausgewählt aus einem oder mehreren aus Cu, Zn, Fe, Al und Mo, umfasst.

4. Flüssige Verbandszusammensetzung zur Verwendung bei der Behandlung oder Prävention einer Infektion und/oder von Wunden nach Anspruch 1 oder Anspruch 2, wobei die Barriere eine hydrophobe Barriere ist.

5. Flüssige Verbandszusammensetzung zur Verwendung bei der Behandlung oder Prävention einer Infektion und/oder von Wunden nach einem der vorangegangenen Ansprüche, wobei die Zusammensetzung dazu in der Lage ist, eine selbsttragende hydrophobe Barriere zu bilden, wenn sie topisch auf ein Individuum aufgetragen wird.

6. Flüssige Verbandszusammensetzung zur Verwendung bei der Behandlung oder Prävention einer Infektion und/oder von Wunden nach einem der vorangegangenen Ansprüche, wobei die Zusammensetzung zur Verwendung bei der Behandlung oder Prävention bakterieller, fungaler, parasitärer oder viraler Infektionen dient, wobei die Infektion gegebenenfalls durch ein gramnegatives oder grampositives Bakterium verursacht wird.

7. Flüssige Verbandszusammensetzung zur Verwendung bei der Behandlung oder Prävention einer Infektion und/oder von Wunden nach Anspruch 6, wobei:
(a) die bakterielle Infektion durch ein Spirochaetes sp., ein Escherichia sp., ein Staphylococcus sp., ein Bacillus sp., ein Pseudomonas sp., ein Vibrio sp., ein Streptococcus sp., ein Klebsiella sp., ein Micrococcus sp., ein Clostridium sp., ein Acinetobacter sp., ein Mycobacterium sp., ein Salmonella sp., ein Chlamydia sp., ein Erysipelothrix sp., ein Corynebacterium sp., ein Leptospira sp., ein Cutibacterium sp, ein Gardnerella sp., ein Campylobacter sp., ein Klebsiella sp., ein Capnocytophaga sp., ein Vibrio sp., ein Pasteurella sp., ein Aeromonas sp., ein Eikenella sp., ein Mycoplasma sp., ein Calymmatobacterium sp., ein Helicobacter sp., ein Clostridium sp., ein Listeria sp., ein Treponema sp., ein Bartonella sp., ein Nocardia sp., ein Yersinia sp., ein Serratia sp., ein Burkholderia sp., ein Actinomyces sp., ein Borrelia sp. oder ein Enterococcus sp. verursacht wird; oder
(b) die fungale Infektion durch ein Candida sp., ein Malassezia sp., ein Trycophyton sp., ein Microsporum sp., ein Epidermophyton sp., ein Fusarium sp., ein Rhizopus sp., ein Rhizomucor sp., ein Mucor sp., ein Syncephalastrum sp., ein Cunninghamella sp., ein Apophysomyces sp., ein Lichtheimia sp., ein Saksenaea sp., ein Aspergillus sp., ein Blastomyces sp., ein Coccidioides sp., ein Cryptococcus sp., ein Histoplasma sp., ein Cochliobolus sp., ein Cladophialophora sp., ein Exophiala sp., ein Madurella sp., ein Pyrenochaeta sp., ein Pneumocystis sp., ein Paracoccidioides sp., ein Sporothrix sp. oder ein Talaromyces sp. Verursacht wird; oder
(c) die parasitäre Infektion durch ein Sarcoptes sp., Leishmania sp., Ancylostomatidae, wie z. B. Ancylostoma sp. oder Necator sp., ein Strongyloides sp., ein Onchocerca sp., Zweiflüglerlarven, Protophormia terraenovae, Cochliomyia hominivorax, Cordylobia anthropophaga oder Sarcophaga bercaea, ein Balamuthia sp. oder Acanthamoeba sp. verursacht wird; oder
(d) die virale Infektion durch ein Papillomavirus, ein Enterovirus, wie z. B. Coxsackievirus, Molluscipoxvirus sp., Varicellovirus oder ein Herpesvirus verursacht wird; oder
(e) die Infektion durch ein Adenoviridae-Virus, ein Caliciviridae-Virus, ein Coronaviridae-Virus, ein Flaviviridae-Virus, ein Herpesviridae-Virus, ein Orthomyxoviridae-Virus, ein Papillomaviridae-Virus, ein Paramyxoviridae-Virus, ein Parvoviridae-Virus, ein Poxviridae-Virus, ein Reoviridae-Virus oder ein Phenuiviridae-Virus verursacht wird.

8. Flüssige Verbandszusammensetzung zur Verwendung bei der Behandlung oder Prävention einer Infektion und/oder von Wunden nach einem der vorangegangenen Ansprüche, wobei die Behandlung das Aufbringen eines zweiten Wirkstoffs auf eine Stelle auf dem Individuum und das Aufbringen der flüssigen Verbandszusammensetzung über die Stelle umfasst, um den zweiten Wirkstoff zu versiegeln, wobei der zweite Wirkstoff ein Antibiotikum, ein antimikrobielles Mittel und/oder einen hämostatischen Wirkstoff umfasst oder wobei der zweite Wirkstoff Iod umfasst.

9. Flüssige Verbandszusammensetzung zur Verwendung bei der Behandlung oder Prävention einer Infektion und/oder von Wunden nach einem der Ansprüche 6 bis 8, wobei die selbsttragende hydrophobe Barriere den Verlust des zweiten Wirkstoffs verglichen mit einer Behandlung ohne Anwendung der selbsttragenden hydrophoben Barriere um zumindest 20 % und gegebenenfalls um zumindest 30 % reduziert.

10. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung zur Behandlung von Kühen, Büffeln, Yaks, Schweinen, Pferden, Schafen, Ziegen, Lamas, Alpakas, Hirschen, Eseln, Zebus, Zebras, Elefanten, Orang-Utans, Schimpansen, Gorillas, Lemuren, Gibbons, Pavianen, Hühnern, Truthähnen, Enten, Emus, Gänsen, Straußen, Katzen, Hunden, Frettchen, Wüstenrennmäusen, Hamstern, Chinchillas, Ratten, Kaninchen, Papageien, Kanarienvögeln, Hühnervögeln, Gänsevögeln oder Sperlingsvögeln dient.

11. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung als Farbzusammensetzung angewendet wird, die gegebenenfalls eine Viskosität bei 25 °C von zwischen 1.000 cP und 5.000 cP aufweist, wobei die flüssige Verbandszusammensetzung gegebenenfalls als Sprühzusammensetzung angewendet wird, die gegebenenfalls eine Viskosität bei 25 °C von zwischen 10 cP und 3.000 cP aufweist.

12. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung zur Behandlung von boviner digitaler Dermatitis, Euterinfektion, Leishmaniose, diabetischen Geschwüren oder Buruli-Geschwüren, Zitzenblockaden, Hornentfernung, Euterdermatitis, nach einer Amputation, Huffäule bei Schafen oder Mauke bei Pferden dient.

13. Flüssige Verbandszusammensetzung zur Verwendung bei der Behandlung oder Prävention einer Infektion und/oder von Wunden nach einem der vorangegangenen Ansprüche, wobei:
(a) der Schellack wachsfreier Schellack, entwachster Schellack, entwachster gebleichter Schellack, entwachster und entfärbter Schellack, Schellackester oder gewachster Schellack ist; und/oder
(b) die Zusammensetzung 10 bis 70 Gew.-% Schellack und gegebenenfalls 40 Gew.- % bis 60 % Gew.-% Schellack umfasst; und/oder
(c) das Lösungsmittel Ethanol, ein Aceton/Wasser-Gemisch, Isopropanol, Propanol und Butanol oder Kombinationen daraus ist; und/oder
(d) der infektionshemmende Wirkstoff auf Metallbasis Kupferchlorid, Kupferacetat, Kupferoxid, Kupferoxohydroxid, Kupferhydroxid, Zinkchlorid, Zinkacetat, Eisenchlorid, Aluminiumchlorid, Molybdänacetat oder Kaolin ist.

14. Flüssige Verbandszusammensetzung zur Verwendung bei der Behandlung oder Prävention einer Infektion und/oder von Wunden nach einem der vorangegangenen Ansprüche, wobei:
(a) der infektionshemmende Wirkstoff auf Metallbasis in einer Konzentration von 20 mMolal bis 500 mMolal vorhanden ist; und/oder
(b) die Zugabe des infektionshemmenden Wirkstoffs auf Metallbasis die Viskosität der Zusammensetzung und/oder die Dicke der Barriere über einer Zusammensetzung, umfassend ausschließlich Schellack, erhöht; und/oder
(c) die antimikrobielle Zusammensetzung gleichzeitig mit einem oder mehreren Antibiotika verabreicht wird, wobei die Zusammensetzung gegebenenfalls an einer Stelle auf das Individuum angewendet wird, auf die das Antibiotikum angewendet wurde; und/oder
(d) die flüssige Verbandszusammensetzung ein Antibiotikum, ein antifungales Mittel und/oder ein Desinfektionsmittel umfasst oder gleichzeitig damit verabreicht wird; und/oder
(e) die Barriere, die gebildet wird, wenn die Zusammensetzung auf ein Individuum angewendet wird, gegenüber Umgebungen mit einem pH-Wert von mehr als pH 9,0 resistent ist; und/oder
(f) die flüssige Verbandszusammensetzung eine weitere Harzkomponente umfasst; und/oder
(g) die flüssige Verbandszusammensetzung weiters Fasern umfasst, vorzugsweise zwischen 1 und 15 Gew.-%, noch bevorzugter zwischen 2,5 und 10 Gew.-% und am bevorzugtesten zwischen 2,5 und 7,5 Gew.-%, wobei die Fasern gegebenenfalls Cellulosefasern sind.

15. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung weiters ein oder mehrere hydrophile Additive als Weichmacher und/oder zum Beschleunigen der Bildung der selbsttragenden hydrophoben Barriere umfasst, wobei die hydrophilen Additive gegebenenfalls aus TEC, einem Polyalkylenglykol, wie z. B. einem Polyethylenglykol, Glycerin, Hexylenglykol und/oder Triacetin ausgewählt sind.

16. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung weiters ein oder mehrere die hydrophobe Barriere verstärkende Mittel umfasst, um die Wasserundurchlässigkeit zu verbessern, wobei das die hydrophobe Barriere der antimikrobiellen Zusammensetzung verstärkende Mittel gegebenenfalls aus Decansäure, Octansäure, Ölsäure und/oder Laurinsäure ausgewählt ist.

17. Flüssige Verbandszusammensetzung zur Verwendung bei der Behandlung oder Prävention einer Infektion und/oder von Wunden nach einem der vorangegangenen Ansprüche, wobei die Zusammensetzung weiters ein oder mehrere Puffermittel umfasst, um den Abbau der Barriere zu verhindern oder zu reduzieren, wobei das Puffermittel gegebenenfalls eine Dicarbonsäure ist, wobei das Dicarboxylat gegebenenfalls Adipinsäure, Azelainsäure, Bernsteinsäure, Pimelinsäure oder Decansäure ist.

18. Flüssige Verbandszusammensetzung zur Verwendung bei der Behandlung oder Prävention einer Infektion und/oder von Wunden nach einem der vorangegangenen Ansprüche, wobei die antimikrobielle Zusammensetzung weiters ein entzündungshemmendes Mittel oder einen Komplexbildner umfasst, um die Beladung oder Löslichkeit des infektionshemmenden Wirkstoffs auf Metallbasis zu verbessern, wobei das entzündungshemmende Mittel oder der Komplexbildner gegebenenfalls aus Salicylsäure, Natriumsalicylat und/oder Propionsäure ausgewählt ist.

19. Flüssige Verbandszusammensetzung zur Verwendung bei der Behandlung oder Prävention einer Infektion und/oder von Wunden nach einem der vorangegangenen Ansprüche, wobei die flüssige Verbandszusammensetzung weiters ein hämostatisches Mittel, wobei das hämostatische Mittel die Gerinnungswirkung verbessert, zur Verwendung in der akuten Wundversorgung umfasst.

20. Flüssige Verbandszusammensetzung zur Verwendung bei der Behandlung oder Prävention einer Infektion und/oder von Wunden nach einem der vorangegangenen Ansprüche, wobei die Zusammensetzung nichtsprühbar ist und eine Tröpfchengröße von 1 mm oder mehr aufweist.

21. Vorrichtung zum Aufbringen einer flüssigen Verbandszusammensetzung zur veterinärmedizinischen Verwendung bei der Behandlung einer Infektion und/oder von Wunden, wobei die Vorrichtung Folgendes umfasst:
eine erste Kammer, die eine Zusammensetzung, umfassend Schellack und einen infektionshemmenden Wirkstoff auf Metallbasis, ausgewählt aus einem oder mehreren aus Cu, Zn, Fe, Al, Mo und Ca, in einem flüchtigen Lösungsmittel, zur Verwendung wie in einem der Ansprüche 1 bis 20 definiert enthält, und eine zweite Kammer, die ein Nicht-Lösungsmittel enthält;
eine Mischkammer in Kommunikation mit der ersten und der zweiten Kammer;
ein Betätigungsmittel zur Abgabe der Zusammensetzung und des Nicht-Lösungsmittels an die Mischkammer, um eine Mischzusammensetzung bereitzustellen, wobei das Nicht-Lösungsmittel Glycerin oder ein Polyalkylenglykol ist; und
einen Applikator in Kommunikation mit der Mischkammer zum Aufbringen der Mischzusammensetzung auf eine Stelle auf einem Individuum, wobei die Zusammensetzung dazu in der Lage ist, eine hydrophobe Barriere zu bilden, wenn diese topisch auf ein Individuum aufgebracht wird, wobei die Zusammensetzung zur veterinärmedizinischen Verabreichung dient und wobei topisch auf ein Individuum aufgebracht keine Anwendung auf die Zähne des Individuums umfasst.

22. Vorrichtung nach Anspruch 21, wobei das Polyalkylenglykol PEG ist und/oder wobei die Vorrichtung ein Dual-Kartuschen-Applikator ist.

23. Unter Druck stehende Sprühabgabevorrichtung, umfassend eine Zusammensetzung, umfassend Schellack und einen Metallwirkstoff in einem flüchtigen Lösungsmittel, zur Verwendung wie in einem der Ansprüche 1 bis 20 definiert und ein Treibmittel zur Abgabe der Zusammensetzung.

## Revendications

1. Composition de pansement liquide pour une utilisation vétérinaire dans le traitement ou la prévention d'une infection et/ou de plaies, dans laquelle la composition comprend de la gomme laque, un actif métallique anti-infectieux choisi parmi un ou plusieurs parmi Cu, Zn, Fe, Al, Mo et Ca, dans laquelle l'actif métallique anti-infectieux est à base de zinc, est choisi parmi un ou plusieurs parmi du chlorure de zinc et des complexes d'acétate de zinc ou de carboxylate de zinc, et un solvant volatil, dans laquelle la composition est capable de former une barrière lorsqu'elle est appliquée par voie topique sur un sujet, dans laquelle la composition est destinée à une administration vétérinaire, et une application par voie topique sur un sujet n'inclut pas une application sur les dents du sujet.

2. Composition de pansement liquide pour utilisation dans le traitement ou la prévention d'une infection et/ou de plaies selon la revendication 1, dans laquelle la composition de pansement liquide est appliquée sur un site d'une plaie ou d'une infection du sujet ou qui est à risque de développer une plaie ou une infection.

3. Composition de pansement liquide pour utilisation dans le traitement ou la prévention d'une infection et/ou de plaies selon la revendication 1 ou la revendication 2, dans laquelle l'actif métallique anti-infectieux comprend un actif métallique anti-infectieux choisi parmi un ou plusieurs parmi Cu, Zn, Fe, Al et Mo.

4. Composition de pansement liquide pour utilisation dans le traitement ou la prévention d'une infection et/ou de plaies selon la revendication 1 ou la revendication 2, dans laquelle la barrière est une barrière hydrophobe.

5. Composition de pansement liquide pour utilisation dans le traitement ou la prévention d'une infection et/ou de plaies selon l'une quelconque des revendications précédentes, dans laquelle la composition est capable de former une barrière hydrophobe autoportante lorsqu'elle est appliquée par voie topique sur un sujet.

6. Composition de pansement liquide pour utilisation dans le traitement ou la prévention d'une infection et/ou de plaies selon l'une quelconque des revendications précédentes, dans laquelle la composition est destinée à une utilisation dans le traitement ou la prévention d'une infection bactérienne, fongique, parasitaire ou virale, facultativement dans laquelle l'infection est causée par une bactérie à Gram négatif ou à Gram positif.

7. Composition de pansement liquide pour utilisation dans le traitement ou la prévention d'une infection et/ou de plaies selon la revendication 6, dans laquelle :
(a) l'infection bactérienne est causée par *Spirochaetes sp., Escherichia sp., Staphylococcus sp., Bacillus sp., Pseudomonas sp., Vibrio sp., Streptococcus sp., Klebsiella sp., Micrococcus sp., Clostridium sp., Acinetobacter sp., Mycobacterium sp., Salmonella sp., Chlamydia sp., Erysipelothrix sp., Corynebacterium sp., Leptospira sp., Cutibacterium sp., Gardnerella sp., Campylobacter sp., Klebsiella sp., Capnocytophaga sp., Vibrio sp., Pasteurella sp., Aeromonas sp., Eikenella sp., Mycoplasma sp., Calymmatobacterium sp., Helicobacter sp.,* Clostrium *sp*., *Listeria sp.,* Trepema *sp., Bartonella sp., Nocardia sp., Yersinia sp., Serratia sp.,* Burkhinia *sp.,* Actomyia *sp., Borrelia sp.,* Enterus *sp*. ; ou
(b) l'infection fongique est causée par *Candida sp., Malassezia sp.,* Trycophyton *sp., Microsporum sp., Epidermophyton sp., Fusarium sp., Rhizopus sp., Rhizomucor sp., Mucor sp., Syncephalastrum sp., Cunninghamella sp.,* Apophysomyces *sp., Lichtheimia sp.,* Saksenaea *sp*., *Aspergillus sp., Blastomyces sp., Coccidioides sp., Cryptococcus sp., Histoplasma sp., Cochliobolus sp., Cladophialophora sp., Exophiala sp., Madurella sp., Pyrenochaeta sp., Pneumocystis sp., Paracoccidioides sp., Sporothrix sp., ou Talaromyces sp.* ; ou
(c) l'infection parasitaire est causée par *Sarcoptes sp., Leishmania sp*., *Ancylostomatidae tels que Ancylostoma sp.* ou *Necator sp., Strongyloides sp., Onchocerca sp.,* des *larves diptères, Protophormia terraenovae, Cochliomyia hominivorax, Cordylobia anthropophage,* ou *Sarcophaga* bercaea, *Balamuthia sp ou Acanthamoeba sp* ; ou
(d) l'infection virale est causée par un *papillomavirus,* un *entérovirus tel que le Coxsackievirus,* le *Molluscipoxvirus sp., un* varicellovirus ou un *herpèsvirus* ; ou
(e) l'infection est causée par un virus Adenoviridae, un virus Caliciviridae, un virus Coronaviridae, un virus Flaviviridae, un virus Herpesviridae, un virus Orthomyxoviridae, un virus Papillomaviridae, un virus Paramyxoviridae, un virus Parvoviridae, un virus Poxviridae, un virus Reoviridae ou un virus Phenuiviridae.

8. Composition de pansement liquide pour utilisation dans le traitement ou la prévention d'une infection et/ou de plaies selon l'une quelconque des revendications précédentes, dans laquelle le traitement comprend l'application d'un second agent actif sur un site sur le sujet et l'application de la composition de pansement liquide sur le site pour bloquer le second agent actif, dans laquelle le second agent actif comprend un agent antibiotique, un agent antimicrobien et/ou un agent actif hémostatique ou dans laquelle le second agent actif comprend de l'iode.

9. Composition de pansement liquide pour utilisation dans le traitement ou la prévention d'une infection et/ou de plaies selon l'une quelconque des revendications 6 à 8, dans laquelle la barrière hydrophobe autoportante réduit la perte du second agent actif d'au moins 20 %, et facultativement d'au moins 30 %, par comparaison à un traitement sans l'application de la barrière hydrophobe autoportante.

10. Composition de pansement liquide pour utilisation dans le traitement ou la prévention d'une infection et/ou de plaies selon l'une quelconque des revendications précédentes, dans laquelle la composition de pansement liquide est destinée au traitement de vaches, de buffles, de yaks, de porcs, de chevaux, de moutons, de chèvres, de lamas, d'alpagas, de cerfs, d'ânes, de zébus, de zèbres, d'éléphants, d'orangs-outans, de chimpanzés, de gorilles, de lémuriens, de gibbons, de babouins, de poulets, de dindes, de canards, d'émeus, d'oies, d'autruches, de chats, de chiens, de furets, de gerbilles, de hamsters, de chinchillas, de rats, de lapins, de perroquets, de canaris, de galliformes, d'ansériformes ou de passeriformes.

11. Composition de pansement liquide pour utilisation dans le traitement ou la prévention d'une infection et/ou de plaies selon l'une quelconque des revendications précédentes, dans laquelle la composition de pansement liquide est appliquée sous forme de composition de peinture, présentant facultativement une viscosité à 25°C comprise entre 1 000 cP et 5 000 cP, facultativement dans laquelle la composition de pansement liquide est appliquée sous forme de composition de pulvérisation, présentant facultativement une viscosité à 25°C comprise entre 10 cP et 3 000 cP.

12. Composition de pansement liquide pour utilisation dans le traitement ou la prévention d'une infection et/ou de plaies selon l'une quelconque des revendications précédentes, dans laquelle la composition de pansement liquide est pour le traitement de la dermatite digitale bovine, de l'infection des mamelles, de la leishmaniose, des ulcères diabétiques ou des ulcères de Buruli, de l'obturation des trayons, de l'enlèvement de la corne, de la dermatite des mamelles, de l'amputation postérieure, de la pourriture des pieds chez les moutons ou de la fièvre de boue chez les chevaux.

13. Composition de pansement liquide pour utilisation dans le traitement ou la prévention d'une infection et/ou de plaies selon l'une quelconque des revendications précédentes, dans laquelle :
(a) la gomme laque est de la gomme laque exempte de cire, de la gomme laque déparaffinée, de la gomme laque blanchie déparaffinée, de la gomme laque déparaffinée et décolorée, de l'ester de gomme laque ou de la gomme laque paraffinée ; et/ou
(b) la composition comprend de 10 à 70 % p/p de gomme laque et facultativement de 40 % p/p à 60 % p/p de gomme laque ; et/ou
(c) le solvant est de l'éthanol, un mélange acétone-eau, de l'isopropanol, du propanol et du butanol, ou des combinaisons de ceux-ci ; et/ou
(d) l'actif métallique anti-infectieux est le chlorure de cuivre, l'acétate de cuivre, l'oxyde de cuivre, l'oxo-hydroxyde de cuivre, l'hydroxyde de cuivre, le chlorure de zinc, l'acétate de zinc, le chlorure de fer, le chlorure d'aluminium, l'acétate de molybdène ou le kaolin.

14. Composition de pansement liquide pour utilisation dans le traitement ou la prévention d'une infection et/ou de plaies selon l'une quelconque des revendications précédentes, dans laquelle :
(a) l'actif métallique anti-infectieux est présent à une concentration de 20 mMolal à 500 mMolal ; et/ou
(b) l'ajout de l'actif métallique anti-infectieux augmente la viscosité de la composition et/ou l'épaisseur de la barrière par rapport à une composition comprenant de la gomme laque seule ; et/ou
(c) la composition antimicrobienne est co-administrée avec un ou plusieurs antibiotiques, la composition étant facultativement appliquée à un sujet à un endroit où l'antibiotique a été appliqué ; et/ou
(d) la composition de pansement liquide comprend ou est co-administrée avec un antibiotique, un agent antifongique et/ou un désinfectant ; et/ou
(e) la barrière formée lorsque la composition est appliquée sur un sujet est résistante à des environnements présentant un pH supérieur à 9,0 ; et/ou
(f) la composition de pansement liquide comprend un composant de résine supplémentaire ; et/ou
(g) la composition de pansement liquide comprend en outre des fibres, de préférence entre 1 et 15 % p/p, plus préférentiellement entre 2,5 et 10 %, le plus préférentiellement entre 2,5 % et 7,5 %, facultativement dans laquelle les fibres sont des fibres de cellulose.

15. Composition de pansement liquide pour utilisation dans le traitement ou la prévention d'une infection et/ou de plaies selon l'une quelconque des revendications précédentes, dans laquelle la composition de pansement liquide comprend en outre un ou plusieurs additifs hydrophiles comme plastifiants et/ou pour accélérer la formation de la barrière hydrophobe autoportante, facultativement dans laquelle les additifs hydrophiles sont choisis parmi TEC, un polyalkylène glycol tel qu'un polyéthylène glycol, le glycérol, l'hexylène glycol et/ou la triacétine.

16. Composition de pansement liquide pour utilisation dans le traitement ou la prévention d'une infection et/ou de plaies selon l'une quelconque des revendications précédentes, dans laquelle la composition de pansement liquide comprend en outre un ou plusieurs agents améliorant la barrière hydrophobe pour améliorer la résistance à l'eau, facultativement dans laquelle l'agent améliorant la barrière hydrophobe de la composition antimicrobienne est choisi parmi l'acide décanoïque, l'acide octanoïque, l'acide oléique et/ou l'acide laurique.

17. Composition de pansement liquide pour utilisation dans le traitement ou la prévention d'une infection et/ou de plaies selon l'une quelconque des revendications précédentes, dans laquelle la composition comprend en outre un ou plusieurs agents tampons pour inhiber ou réduire la dégradation de la barrière, facultativement dans laquelle l'agent tampon est un acide di-carboxylique, facultativement dans laquelle le dicarboxylate est l'acide adipique, l'acide azélaïque, l'acide succinique, l'acide pimélique ou l'acide décanoïque.

18. Composition de pansement liquide pour utilisation dans le traitement ou la prévention d'une infection et/ou de plaies selon l'une quelconque des revendications précédentes, dans laquelle la composition antimicrobienne inclut en outre un agent anti-inflammatoire ou un agent complexant pour améliorer la charge ou la solubilité de l'actif métallique anti-infectieux, facultativement dans laquelle l'agent anti-inflammatoire ou l'agent complexant est choisi parmi l'acide salicylique, le salicylate de sodium et/ou l'acide propionique.

19. Composition de pansement liquide pour utilisation dans le traitement ou la prévention d'une infection et/ou de plaies selon l'une quelconque des revendications précédentes, dans laquelle la composition de pansement liquide comprend en outre un agent hémostatique, dans laquelle l'agent hémostatique améliore l'action de coagulation pour utilisation dans de soins de plaies aiguës.

20. Composition de pansement liquide pour utilisation dans le traitement ou la prévention d'une infection et/ou de plaies selon l'une quelconque des revendications précédentes, dans laquelle la composition est non pulvérisable et présente des tailles de gouttelette de 1 mm ou plus.

21. Dispositif pour appliquer une composition de pansement liquide pour une utilisation vétérinaire dans le traitement d'une infection et/ou de plaies, le dispositif comprenant :
une première chambre contenant une composition comprenant de la gomme laque et un actif métallique anti-infectieux choisi parmi Cu, Zn, Fe, Al, Mo et Ca dans un solvant volatil pour une utilisation telle que définie dans l'une quelconque des revendications 1 à 20 et une seconde chambre contenant un non-solvant ;
une chambre de mélange en communication avec les première et seconde chambres ;
des moyens d'actionnement pour délivrer la composition et le non-solvant à la chambre de mélange pour fournir une composition mixte, dans laquelle le non-solvant est du glycérol ou un polyalkylène glycol ; et
un applicateur en communication avec la chambre de mélange pour appliquer la composition mixte à un emplacement sur un sujet, dans lequel la composition est capable de former une barrière hydrophobe lorsqu'elle est appliquée par voie topique sur un sujet, dans lequel la composition est pour une administration vétérinaire et dans lequel l'application par voie topique sur un sujet n'inclut pas une application sur les dents du sujet.

22. Dispositif selon la revendication 21, dans lequel le polyalkylène glycol est du PEG, et/ou dans lequel le dispositif est un applicateur à double cartouche.

23. Dispositif de distribution par pulvérisation sous pression comprenant une composition comprenant de la gomme laque et un actif métallique dans un solvant volatil pour une utilisation selon l'une quelconque des revendications 1 à 20 et un agent propulseur pour délivrer la composition.
